# EUROPEAN PATENT SPECIFICATION

(11) **EP 3 934 635 B1**
(45) Date of publication and mention of the grant of the patent: **20.05.2026**
(21) Application number: 20767314.6
(22) Date of filing: 04.03.2020
(51) Int. Cl.: A61K 31/165, A61K 31/24, A61P 25/00, A61K 31/245, A61K 38/00, C07K 5/062

(54) **COMPOSITIONS COMPRISING ENZYME-CLEAVABLE AMPHETAMINE PRODRUGS AND INHIBITORS THEREOF**
ZUSAMMENSETZUNGEN AUS ENZYMSPALTBAREN AMPHETAMIN-PRODRUGS UND INHIBITOREN DAVON
COMPOSITIONS COMPRENANT DES PROMÉDICAMENTS D'AMPHÉTAMINES CLIVABLES PAR ENZYME ET LEURS INHIBITEURS

(30) Priority: 06.03.2019 US 201962814630 P
(43) Date of publication of application: 12.01.2022
(73) Proprietor: Ensysce Biosciences, Inc., La Jolla, CA 92037 (US); Jenkins, Thomas E., Palo Alto, CA 94303 (US); Husfeld, Craig O., Palo Alto, CA 94303 (US); Seroogy, Julie D., Palo Alto, CA 94303 (US); Wray, Jonathan W., Palo Alto, CA 94303 (US)
(72) Inventor: JENKINS, Thomas E., Palo Alto, California 94303 (US); HUSFELD, Craig O., Palo Alto, California 94303 (US); SEROOGY, Julie D., Palo Alto, California 94303 (US); WRAY, Jonathan W., Palo Alto, California 94303 (US)
(74) Representative: Mewburn Ellis LLP
(86) International application number: PCT/US2020/021016
(87) International publication number: WO 2020/181000

(56) References cited:
- EP-A2- 2 433 655
- WO-A1-2008/101202
- WO-A1-2011/133347
- WO-A1-2011/133348
- WO-A2-2008/101187
- WO-A2-2012/122422
- US-A1- 2005 054 561
- US-B2- 8 497 237
- US-B2- 8 614 346
- PERONA ET AL.: "Structural basis of substrate specificity in the serine proteases", PROTEIN SCIENCE, vol. 4, 1995, pages 337 - 360, XP002064763

## Description

### Technical Field

The present invention relates an amphetamine-arginine-glycine-acetate compound (Compound AM-9) and an amphetamine-arginine-alanine-acetate compound (Compound AM10), and compositions thereof. The present invention also relates to the aforementioned compounds and compositions for use in medical therapy or in the treatment or prevention of attention deficit disorder (ADHD).

### Introduction

Amphetamines are susceptible to misuse, abuse, or overdose. Use of and access to these drugs therefore needs to be controlled. The control of access to the drugs is expensive to administer and can result in denial of treatment for patients that are not able to present themselves for dosing. Furthermore, control of use is often ineffective, leading to substantial morbidity and deleterious social consequences.

WO 2011/133348 A1 - PHARMACOFORE, INC., et al. describes pharmaceutical compositions comprising certain amphetamine prodrugs of the following structural formula, which may optionally further comprise an enzyme inhibitor:

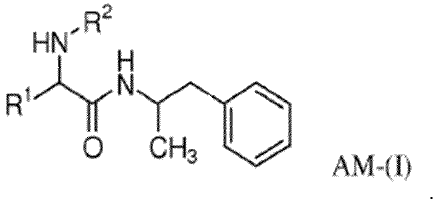

WO 2011/133347 A1 - PHARMACOFORE, INC., et al. describes pharmaceutical compositions comprising trypsin-cleavable amphetamine prodrugs.

WO 2012/122422 A2 - SIGNATURE THERAPEUTICS INC., et al. describes certain prodrug compounds of, e.g., Formula (I) below and compositions comprising such compounds and optionally a trypsin inhibitor:

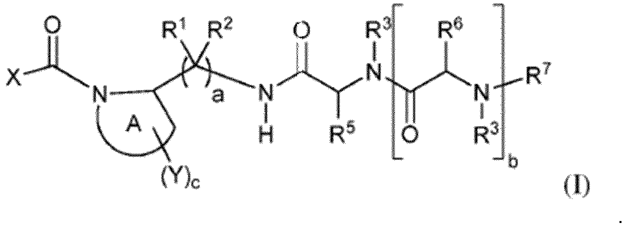

WO 2008/101202 A1 - PHARMACOFORE, INC., et al. describes certain N-17-alkylated prodrugs of opiods such as the compound shown below:

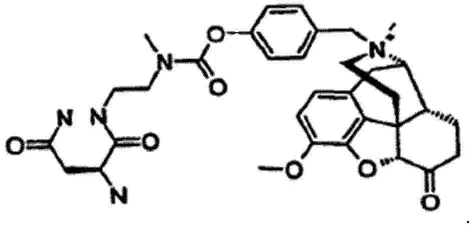

US 2011/105381 A2 - PHARMACOFORE, INC. describes certain compounds of formula (I), below, which are allegedly useful as prodrugs of peripheral phenolic opioid antagonists:

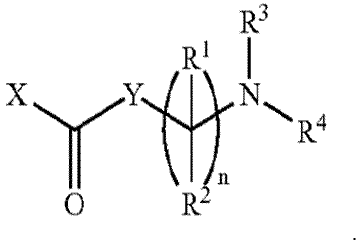

### Summary

The embodiments provide Compound AM-9 (Amphetamine-arginine-glycine-acetate), shown below: 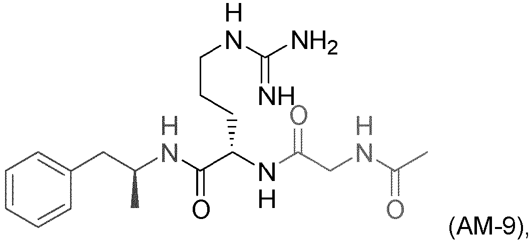 or acceptable salts, solvates, and hydrates thereof. Compound AM-9 is a prodrug that provides controlled immediate release of amphetamine.

The embodiments also provide Compound AM-10 (Amphetamine-arginine-alanine-acetate), shown below: 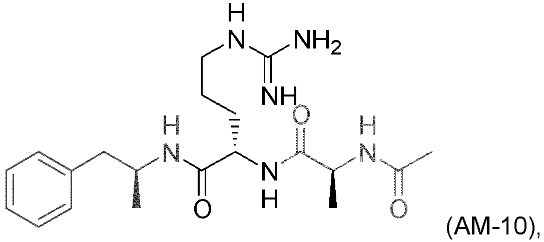 or acceptable salts, solvates, and hydrates thereof. Compound AM-10 is a prodrug that provides controlled immediate release of amphetamine.

The embodiments provide a composition, which comprises one or more of Compound AM-9 and Compound AM-10 or pharmaceutically acceptable salts, solvates, and hydrates thereof.

The disclosure provides Compound AM-9 and Compound AM-10, amphetamine prodrugs that provides controlled immediate release of amphetamine, each having an enzyme-cleavable moiety such that Compound AM-9 and Compound AM-10 provide controlled immediate release of ampethamine via enzyme cleavage. Compound AM-9 and Compound AM-10 provide efficient delivery of amphetamine when ingested.

The present disclosure also provides pharmaceutical compositions, and their methods of use, where the pharmaceutical compositions comprise one or more amphetamine prodrug, Compound AM-9 and Compound AM-10 or pharmaceutically acceptable salts, solvates, and hydrates thereof, that provides controlled release of amphetamine via enzyme cleavage. Such compositions can optionally provide an inhibitor, such as a trypsin inhibitor, that interacts with the enzyme that mediates the controlled release of amphetamine from the prodrug so as to attenuate enzymatic cleavage of the prodrug. The disclosure provides for the enzyme being a gastrointestinal (GI) enzyme, such as trypsin.

### Brief Description of the Figures

Figure 1 compares mean plasma concentrations over time of amphetamine following oral (PO) administration of Compound AM-9 to rats according to one embodiment.
Figure 2 compares mean plasma concentrations over time of amphetamine release following PO administration of different doses of Compound AM-9 to rats according to one embodiment.
Figure 3 compares mean plasma concentrations over time of analyte following PO administration of Compound AM-9 to rats according to one embodiment.
Figure 4 compares mean plasma concentrations over time of amphetamine following PO administration of 1) d-amphetamine; 2) Compound AM-9 and 3) Vyvanse to rats according to one embodiment.
Figure 5 compares mean plasma concentrations over time of amphetamine following PO administration of amphetamine and Compound AM-9 to dogs according to one embodiment.
Figure 6 compares mean plasma concentrations over time of analyte following PO administration of Compound AM-9 to rats according to one embodiment.
Figure 7 compares mean plasma concentrations over time of amphetamine following PO administration of 1) d-amphetamine; 2) Compound AM-9 and 3) Vyvanse to dogs according to one embodiment.
Figures 8A and 8B compare mean plasma concentrations over time of amphetamine following PO administration of Compound AM-9 to rats in the absence and presence of trypsin inhibitor nafamostat according to one embodiment.
Figure 9 compares mean plasma concentrations over time of amphetamine following PO administration of Compound AM-9 to rats in the absence and presence of trypsin inhibitor nafamostat according to another embodiment.
Figure 10 compares mean plasma concentrations over time of Compound AM-9 and the resulting mean plasma concentrations of amphetamine over time following IV administration of Compound AM-9 to rats according to one embodiment.
Figure 11 compares mean plasma concentrations over time and the mean CSF concentrations over time of Compound AM-9 following IV administration of Compound AM-9 to rats according to one embodiment.
Figure 12 compares mean plasma concentrations over time of amphetamine following PO administration of Compound AM-10 to rats according to one embodiment.
Figure 13 compares mean plasma concentrations over time of amphetamine release following PO administration of different doses of Compound AM-10 to rats.

### Terms

The following terms have the following meaning unless otherwise indicated. Any undefined terms have their art recognized meanings.

"Dose unit" as used herein refers to a combination of a trypsin-cleavable prodrug (e.g., trypsin-cleavable prodrug) and a trypsin inhibitor. A "single dose unit" is a single unit of a combination of a trypsin-cleavable prodrug (e.g., trypsin-cleavable prodrug) and a trypsin inhibitor, where the single dose unit provide a therapeutically effective amount of drug (i.e., a sufficient amount of drug to effect a therapeutic effect, e.g., a dose within the respective drug's therapeutic window, or therapeutic range). "Multiple dose units" or "multiples of a dose unit" or a "multiple of a dose unit" refers to at least two single dose units.

"Gastrointestinal enzyme" or "GI enzyme" refers to an enzyme located in the gastrointestinal (GI) tract, which encompasses the anatomical sites from mouth to anus. Trypsin is an example of a GI enzyme.

"Gastrointestinal enzyme-cleavable moiety" or "GI enzyme-cleavable moiety" refers to a group comprising a site susceptible to cleavage by a GI enzyme. For example, a "trypsin-cleavable moiety" refers to a group comprising a site susceptible to cleavage by trypsin.

"Gastrointestinal enzyme inhibitor" or "GI enzyme inhibitor" refers to any agent capable of inhibiting the action of a gastrointestinal enzyme on a substrate. The term also encompasses salts of gastrointestinal enzyme inhibitors. For example, a "trypsin inhibitor" refers to any agent capable of inhibiting the action of trypsin on a substrate.

"Patient" includes humans, and also other mammals, such as livestock, zoo animals and companion animals, such as a cat, dog or horse.

"Pharmaceutical composition" refers to at least one compound and can further comprise a pharmaceutically acceptable carrier, with which the compound is administered to a patient.

"Pharmaceutically acceptable carrier" refers to a diluent, adjuvant, excipient or vehicle with, or in which a compound is administered.

"Pharmaceutically acceptable salt" refers to a salt of a compound, which possesses the desired pharmacological activity of the compound. Such salts include: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl) benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2.2.2]-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or (2) salts formed when an acidic proton present in the compound is replaced by a metal ion, e.g., an alkali metal ion, an alkaline earth ion, or an aluminum ion; or coordinates with an organic base such as ethanolamine, diethanolamine, triethanolamine, N-methylglucamine and the like.

"Pharmacodynamic (PD) profile" refers to a profile of the efficacy of a drug in a patient (or subject or user), which is characterized by PD parameters. "PD parameters" include "drug Emax" (the maximum drug efficacy),"drug EC50" (the concentration of drug at 50% of the Emax) and side effects.

"PK parameter" refers to a measure of drug concentration in blood or plasma, such as: 1) "drug Cmax", the maximum concentration of drug achieved in blood or plasma; 2) "drug Tmax", the time elapsed following ingestion to achieve Cmax; and 3) "drug exposure", the total concentration of drug present in blood or plasma over a selected period of time, which can be measured using the area under the curve (AUC) of a time course of drug release over a selected period of time (t). Modification of one or more PK parameters provides for a modified PK profile.

"PK profile" refers to a profile of drug concentration in blood or plasma. Such a profile can be a relationship of drug concentration over time (i.e., a "concentration-time PK profile") or a relationship of drug concentration versus number of doses ingested (i.e., a "concentration-dose PK profile"). A PK profile is characterized by PK parameters.

"Preventing" or "prevention" or "prophylaxis" refers to a reduction in risk of occurrence of a condition, such as pain.

"Prodrug" refers to a derivative of an active agent that requires a transformation within the body to release the active agent. In certain embodiments, the transformation is an enzymatic transformation. Prodrugs are frequently, although not necessarily, pharmacologically inactive until converted to the active agent.

"Promoiety" refers to a form of protecting group that, when used to mask a functional group within an active agent, converts the active agent into a prodrug. Typically, the promoiety will be attached to the drug *via* bond(s) that are cleaved by enzymatic or non-enzymatic means *in vivo.*

"Solvate" as used herein refers to a complex or aggregate formed by one or more molecules of a solute, e.g. a prodrug or a pharmaceutically-acceptable salt thereof, and one or more molecules of a solvent. Such solvates are typically crystalline solids having a substantially fixed molar ratio of solute and solvent. Representative solvents include by way of example, water, methanol, ethanol, isopropanol, acetic acid, and the like. When the solvent is water, the solvate formed is a hydrate.

"Therapeutically effective amount" means the amount of a compound (e.g., prodrug) that, when administered to a patient for preventing or treating a condition such as pain, is sufficient to effect such treatment. The "therapeutically effective amount" will vary depending on the compound, the condition and its severity and the age, weight, *etc.,* of the patient.

"Treating" or "treatment" of any condition, such as pain, refers, in certain embodiments, to ameliorating the condition (i.e., arresting or reducing the development of the condition). In certain embodiments "treating" or "treatment" refers to ameliorating at least one physical parameter, which may not be discernible by the patient. In certain embodiments, "treating" or "treatment" refers to inhibiting the condition, either physically, (e.g., stabilization of a discernible symptom), physiologically, (e.g., stabilization of a physical parameter), or both. In certain embodiments, "treating" or "treatment" refers to delaying the onset of the condition.

### Detailed Description

Before the present invention is further described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Any references herein to methods of treatment by therapy or surgery or *in vivo* diagnosis are to be interpreted as references to compounds, pharmaceutical compositions and medicaments described herein for use in those methods.

It must be noted that as used herein and in the appended claims, the singular forms "a," "an," and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

It should be understood that as used herein, the term "a" entity or "an" entity refers to one or more of that entity. For example, a compound refers to one or more compounds. As such, the terms "a", "an", "one or more" and " at least one" can be used interchangeably. Similarly the terms "comprising", "including" and "having" can be used interchangeably.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described.

Except as otherwise noted, the methods and techniques of the present embodiments are generally performed according to conventional methods well known in the art and as described in various general and more specific references that are cited and discussed throughout the present specification. See, e.g., Loudon, Organic Chemistry, Fourth Edition, New York: Oxford University Press, 2002, pp. 360-361, 1084-1085; Smith and March, March's Advanced Organic Chemistry: Reactions, Mechanisms, and Structure, Fifth Edition, Wiley-Interscience, 2001.

The nomenclature used herein to name the subject compounds is illustrated in the Examples herein. In certain instances, this nomenclature is derived using the commercially-available AutoNom software (MDL, San Leandro, Calif.).

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination. All combinations of the embodiments pertaining to the chemical groups represented by the variables are specifically embraced by the present invention and are disclosed herein just as if each and every combination was individually and explicitly disclosed, to the extent that such combinations embrace compounds that are stable compounds (i.e., compounds that can be isolated, characterised, and tested for biological activity). In addition, all sub-combinations of the chemical groups listed in the embodiments describing such variables are also specifically embraced by the present invention and are disclosed herein just as if each and every such sub-combination of chemical groups was individually and explicitly disclosed herein.

### General Synthetic Procedures

Many general references providing commonly known chemical synthetic schemes and conditions useful for synthesizing the disclosed compounds are available (see, e.g., Smith and March, March's Advanced Organic Chemistry: Reactions, Mechanisms, and Structure, Fifth Edition, Wiley-Interscience, 2001; or Vogel, A Textbook of Practical Organic Chemistry, Including Qualitative Organic Analysis, Fourth Edition, New York: Longman, 1978).

Compounds as described herein can be purified by any of the means known in the art, including chromatographic means, such as high performance liquid chromatography (HPLC), preparative thin layer chromatography, flash column chromatography and ion exchange chromatography. Any suitable stationary phase can be used, including normal and reversed phases as well as ionic resins. See, e.g., Introduction to Modern Liquid Chromatography, 2nd Edition, ed. L. R. Snyder and J. J. Kirkland, John Wiley and Sons, 1979; and Thin Layer Chromatography, ed E. Stahl, Springer-Verlag, New York, 1969.

During any of the processes for preparation of the compounds of the present disclosure, it may be necessary and/or desirable to protect sensitive or reactive groups on any of the molecules concerned. This can be achieved by means of conventional protecting groups as described in standard works, such as T. W. Greene and P. G. M. Wuts, "Protective Groups in Organic Synthesis", Fourth edition, Wiley, New York 2006. The protecting groups can be removed at a convenient subsequent stage using methods known from the art.

The compounds described herein can contain one or more chiral centers and/or double bonds and therefore, can exist as stereoisomers, such as double-bond isomers (i.e., geometric isomers), enantiomers or diastereomers. Accordingly, all possible enantiomers and stereoisomers of the compounds including the stereoisomerically pure form (e.g., geometrically pure, enantiomerically pure or diastereomerically pure) and enantiomeric and stereoisomeric mixtures are included in the description of the compounds herein. Enantiomeric and stereoisomeric mixtures can be resolved into their component enantiomers or stereoisomers using separation techniques or chiral synthesis techniques well known to the skilled artisan. The compounds can also exist in several tautomeric forms including the enol form, the keto form and mixtures thereof. Accordingly, the chemical structures depicted herein encompass all possible tautomeric forms of the illustrated compounds.

The compounds described also include isotopically labeled compounds where one or more atoms have an atomic mass different from the atomic mass conventionally found in nature. Examples of isotopes that can be incorporated into the compounds disclosed herein include, but are not limited to, ²H, ³H, ¹¹C, ¹³C, ¹⁴C, ¹⁵N, ¹⁸O, ¹⁷O, etc. Compounds can exist in unsolvated forms as well as solvated forms, including hydrated forms. In general, compounds can be hydrated or solvated. Certain compounds can exist in multiple crystalline or amorphous forms. In general, all physical forms are equivalent for the uses contemplated herein and are intended to be within the scope of the present disclosure.

### Amphetamine Prodrugs

Amphetamine refers to a chemical substance that exerts its pharmacological action by modulating neurotransmitters, such as dopamine, serotonin and norepinephrine. The disclosure provides for an amphetamine prodrug, wherein amphetamine has the following general structure:

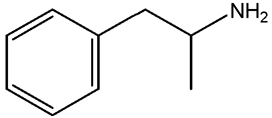

In certain embodiments, amphetamine is a compound with a pharmacophore that crosses the blood-brain barrier and has CNS stimulation and central appetite suppressant effects. See, for example, Foye's Principles of Medicinal Chemistry, Sixth Edition, ed. T.L. Lemke and D.A. Williams, Lippincott Williams & Wilkins, 2008, particularly Chapter 13, pages 392-416.

The present disclosure provides an amphetamine prodrug which provides enzymatically-controlled release of amphetamine. The disclosure provides a promoiety that is attached to amphetamine through the amphetamine amino group.

Reference will now be made in detail to various embodiments. It will be understood that the invention is not limited to these embodiments.

The embodiments provide Compound AM-9 (Amphetamine-arginine-glycine-acetate), shown below: 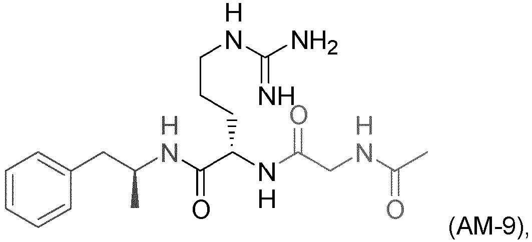 or acceptable salts, solvates, and hydrates thereof.

The embodiments provide Compound AM-10 (Amphetamine-arginine-alanine-acetate), shown below: 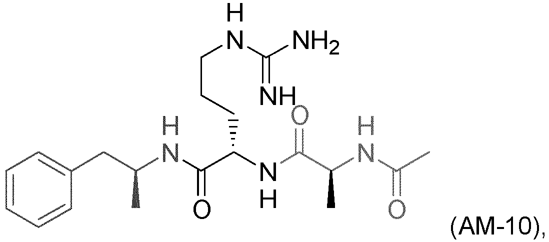 or acceptable salts, solvates, and hydrates thereof. Compound AM-10 is a prodrug that provides controlled immediate release of amphetamine.

The embodiments provide a composition, which comprises one or more of Compound AM-9 and Compound AM-10 or pharmaceutically acceptable salts, solvates, and hydrates thereof.

The disclosure provides Compound AM-9 and Compound AM-10, amphetamine prodrugs that provides controlled immediate release of amphetamine, each having an enzyme-cleavable moiety such that Compound AM-9 and Compound AM-10 provide controlled immediate release of ampethamine via enzyme cleavage. Compound AM-9 and Compound AM-10 provide efficient delivery of amphetamine when ingested.

In Compound AM-9 and Compound AM-10, the enzyme capable of cleaving the enzyme-cleavable moiety may be a peptidase, also referred to as a protease - the promoiety comprising the enzyme-cleavable moiety being linked to the nucleophilic nitrogen through an amide (e.g. a peptide: -NHC(O)-) bond. In some embodiments, the enzyme is a digestive enzyme of a protein. The disclosure provides for the enzyme being a GI enzyme, such as trypsin and for the enzyme-cleavable moiety being a GI enzyme-cleavable moiety, such as a trypsin-cleavable moiety.

The corresponding prodrug provides post administration-activated, controlled release of amphetamine. The prodrug requires enzymatic cleavage to initiate release of amphetamine, and thus the rate of release of amphetamine depends upon the rate of enzymatic cleavage. Compound AM-9 and Compound AM-10 provide efficient controlled immediate release of amphetamine due to a rapid enzyme cleavage rate. The prodrug is configured so that it will not provide excessively high plasma levels of the active drug if it is administered inappropriately, and cannot readily be decomposed to afford the active drug other than by enzymatic cleavage.

In embodiments, the subject compounds, Compound AM-9 (Amphetamine-arginine-glycine-acetate) and Compound AM-10 (Amphetamine-arginine-alanine-acetate) provide for immediate release of amphetamine when administered to a subject. In some embodiments, the subject compounds provide for a peak plasma concentration of amphetamine that is within 60 minutes or less of administering the compound to a subject, such as 50 minutes or less, such as 45 minutes or less, such as 30 minutes or less, such as 20 minutes or less, such as 15 minutes or less, such as 10 minutes or less and including providing for a peak plasma concentration of amphetamine that is within 5 minutes or less of administering the compound to the subject. In one example, administration of Compound AM-9 provides for a peak plasma concentration of amphetamine in a subject within 5 to 60 minutes of administration, such as from 10 minute to 50 minutes, such as from 15 minutes to 45 minutes and including from 20 minutes to 30 minutes. In another example, administration of Compound AM-10 provides for a peak plasma concentration of amphetamine in a subject within 5 to 60 minutes of administration, such as from 10 minute to 50 minutes, such as from 15 minutes to 45 minutes and including from 20 minutes to 30 minutes.

### General Synthetic Procedures for Compounds

Representative synthetic schemes for compounds disclosed herein are shown below. Compound AM-9 and Compound AM-10 can be synthesized by using the disclosed methods.

The promoieties described herein, may be prepared and attached to compounds containing amino groups by procedures known to those of skill in the art (See e.g., Green et al., "Protective Groups in Organic Chemistry," (Wiley, 2nd ed. 1991); Harrison et al., "Compendium of Synthetic Organic Methods," Vols. 1 8 (John Wiley and Sons, 1971 1996); "Beilstein Handbook of Organic Chemistry," Beilstein Institute of Organic Chemistry, Frankfurt, Germany; Feiser et al., "Reagents for Organic Synthesis," Volumes 1 17, (Wiley Interscience); Trost et al., "Comprehensive Organic Synthesis," (Pergamon Press, 1991); "Theilheimer's Synthetic Methods of Organic Chemistry," Volumes 1 45, (Karger, 1991); March, "Advanced Organic Chemistry," (Wiley Interscience), 1991; Larock "Comprehensive Organic Transformations," (VCH Publishers, 1989); Paquette, "Encyclopedia of Reagents for Organic Synthesis," (John Wiley & Sons, 1995), Bodanzsky, "Principles of Peptide Synthesis," (Springer Verlag, 1984); Bodanzsky, "Practice of Peptide Synthesis," (Springer Verlag, 1984). Further, starting materials may be obtained from commercial sources or via well established synthetic procedures, supra.

To synthesize Compound AM-9 and AM-10, 1-phenylpropan-2-amine is first coupled with Boc-Arg(Pbf)-OH. Standard peptide coupling reagents can be used for this reaction. For example, suitable peptide coupling reagents include, but are not limited to, EDCI and HOBt, PyBroP and diisopropylethylamine, or HATU. Then, the Boc group is removed. The Boc group can be removed with acidic conditions. For example, suitable reagents that can be used for the deprotection reaction include trifluoroacetic acid and hydrochloric acid.

A di-aminoacid or peptide group (Arg-Gly for Compound AM-9 or Arg-Ala for Compound AM-10) is attached. Reaction can be aided with use of activation reagents, such as symmetric anhydrides, O-(benzotriazol-1-yl)-N,N,N',N'-tetramethyluronium hexafluorophosphate (HBTU), dicyclohexylcarbodiimide (DCC) diisopropylcarbodiimide (DIC)/1-hydroxybenzotriazole (HOBt), and benzotriazole-1-yl oxytris(dimethylamino)phosphonium hexafluorophosphate (BOP).

Then, the Pbf group is removed to yield Compound AM-9 or Compound AM-10. The Pbf group and can be removed with acidic conditions. For example, a suitable reagent that can be used for the deprotection reaction is trifluoroacetic acid.

### Trypsin Inhibitors

As disclosed herein, the present disclosure also provides pharmaceutical compositions, and their methods of use, where the pharmaceutical compositions comprise a prodrug, Compound AM-9 and Compound AM-10, that provides controlled release of amphetamine via enzyme cleavage, and a trypsin inhibitor that interacts with the enzyme that mediates the enzymatically-mediated release of amphetamine from the prodrug so as to attenuate enzymatic cleavage of the prodrug. Such disclosure provides for the enzyme being trypsin.

As used herein, the term "trypsin inhibitor" refers to any agent capable of inhibiting the action of trypsin on a substrate. The term "trypsin inhibitor" also encompasses salts of trypsin inhibitors. The ability of an agent to inhibit trypsin can be measured using assays well known in the art. For example, in a typical assay, one unit corresponds to the amount of inhibitor that reduces the trypsin activity by one benzoyl-L-arginine ethyl ester unit (BAEE-U). One BAEE-U is the amount of enzyme that increases the absorbance at 253 nm by 0.001 per minute at pH 7.6 and 25°C. See, for example, K. Ozawa, M. Laskowski, 1966, J. Biol. Chem. 241, 3955 and Y. Birk, 1976, Meth. Enzymol. 45, 700. In certain instances, a trypsin inhibitor can interact with an active site of trypsin, such as the S1 pocket and the S3/4 pocket. The S1 pocket has an aspartate residue which has affinity for a positively charged moiety. The S3/4 pocket is a hydrophobic pocket. The disclosure provides for specific trypsin inhibitors and non-specific serine protease inhibitors.

There are many trypsin inhibitors known in the art, both those specific to trypsin and those that inhibit trypsin and other proteases such as chymotrypsin. The disclosure provides for trypsin inhibitors that are proteins, peptides, and small molecules. The disclosure provides for trypsin inhibitors that are irreversible inhibitors or reversible inhibitors. The disclosure provides for trypsin inhibitors that are competitive inhibitors, non-competitive inhibitors, or uncompetitive inhibitors. The disclosure provides for natural, synthetic or semi-synthetic trypsin inhibitors.

Trypsin inhibitors can be derived from a variety of animal or vegetable sources: for example, soybean, corn, lima and other beans, squash, sunflower, bovine and other animal pancreas and lung, chicken and turkey egg white, soy-based infant formula, and mammalian blood. Trypsin inhibitors can also be of microbial origin: for example, antipain; see, for example, H. Umezawa, 1976, Meth. Enzymol. 45, 678. A trypsin inhibitor can also be an arginine or lysine mimic or other synthetic compound: for example arylguanidine, benzamidine, 3,4-dichloroisocoumarin, diisopropylfluorophosphate, gabexate mesylate, phenylmethanesulfonyl fluoride, or substituted versions or analogs thereof. In certain embodiments, trypsin inhibitors comprise a covalently modifiable group, such as a chloroketone moiety, an aldehyde moiety, or an epoxide moiety. Other examples of trypsin inhibitors are aprotinin, camostat and pentamidine.

As used herein, an arginine or lysine mimic is a compound that is capable of binding to the P¹ pocket of trypsin and/or interfering with trypsin active site function. The arginine or lysine mimic can be a cleavable or non-cleavable moiety.

In one embodiment, the trypsin inhibitor is derived from soybean. Trypsin inhibitors derived from soybean (*Glycine max*) are readily available and are considered to be safe for human consumption. They include, but are not limited to, SBTI, which inhibits trypsin, and Bowman-Birk inhibitor, which inhibits trypsin and chymotrypsin. Such trypsin inhibitors are available, for example from Sigma-Aldrich, St. Louis, MO, USA.

It will be appreciated that the pharmaceutical composition according to the embodiments may further comprise one or more other trypsin inhibitors.

As stated above, a trypsin inhibitor can be an arginine or lysine mimic or other synthetic compound. In certain embodiments, the trypsin inhibitor is an arginine mimic or a lysine mimic, wherein the arginine mimic or lysine mimic is a synthetic compound.

Certain trypsin inhibitors include compounds of formula: 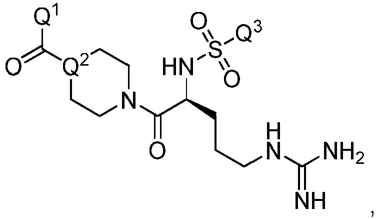 wherein:
Q¹ is selected from -O-Q⁴ or -Q⁴-COOH, where Q⁴ is C₁-C₄ alkyl;
Q² is N or CH; and
Q³ is aryl or substituted aryl.

Certain trypsin inhibitors include compounds of formula: 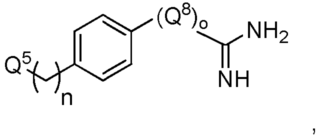 wherein:
Q⁵ is -C(O)-COOH or -NH-Q⁶-Q⁷-SO₂-C₆H₅, where
Q⁶ is -(CH₂)ₚ-COOH;
Q⁷ is -(CH₂)ᵣ-C₆H₅;
Q⁸ is NH;
n is a number from zero to two;
o is zero or one;
p is an integer from one to three; and
r is an integer from one to three.

Certain trypsin inhibitors include compounds of formula: 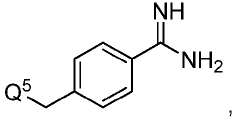 wherein:
Q⁵ is -C(O)-COOH or -NH-Q⁶-Q⁷-SO₂-C₆H₅, where
Q⁶ is -(CH₂)ₚ-COOH;
Q⁷ is -(CH₂)ᵣ-C₆H₅; and
p is an integer from one to three; and
r is an integer from one to three.

Certain trypsin inhibitors include the following:

| | | |
|---|---|---|
| Compound 101 | 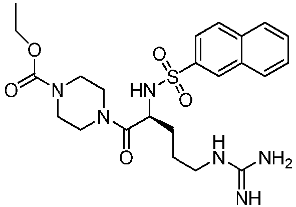 | (S)-ethyl 4-(5-guanidino-2-(naphthalene-2-sulfonamido)pentanoyl)p iperazine-1-carboxylate |
| Compound 102 | 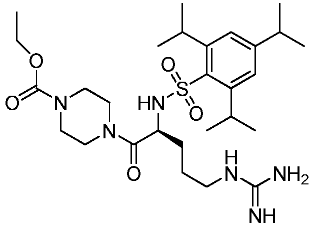 | (S)-ethyl 4-(5-guanidino-2-(2,4,6-triisopropylphenylsulfona mido)pentanoyl)piperazi ne-1-carboxylate |
| Compound 103 | 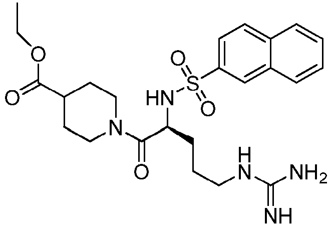 | (S)-ethyl 1-(5-guanidino-2-(naphthalene-2-sulfonamido)pentanoyl)p iperidine-4-carboxylate |
| Compound 104 | 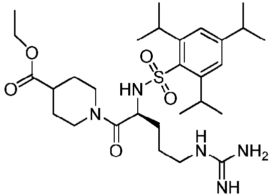 | (S)-ethyl 1-(5-guanidino-2-(2,4,6-triisopropylphenylsulfona mido)pentanoyl)piperidin e-4-carboxylate |
| Compound 105 | 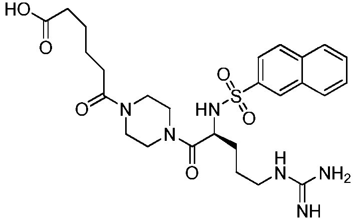 | (S)-6-(4-(5-guanidino-2-(naphthalene-2-sulfonamido)pentanoyl)p iperazin-1-yl)-6-oxohexanoic acid |
| Compound 106 | 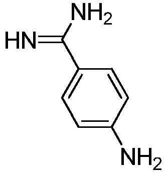 | 4-aminobenzimidamide (also 4-aminobenzamidine) |
| Compound 107 | 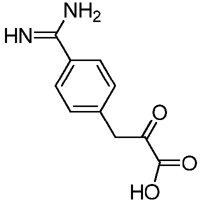 | 3-(4-carbamimidoylphenyl)-2-oxopropanoic acid |
| Compound 108 | 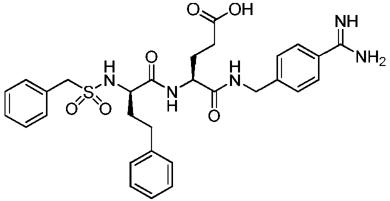 | (S)-5-(4-carbamimidoylbenzylami no)-5-oxo-4-((R)-4-phenyl-2-(phenylmethylsulfonamid o)butanamido)pentanoic acid |
| Compound 109 | 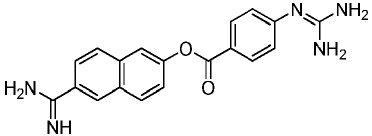 | 6-carbamimidoylnaphthale n-2-yl 4-(diaminomethyleneamin o)benzoate |
| Compound 110 | 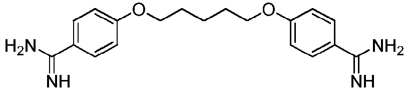 | 4,4'-(pentane-1,5-diylbis(oxy))dibenzimida mide |

A description of methods to prepare Compound 101, Compound 102, Compound 103, Compound 104, Compound 105, Compound 107, and Compound 108 is provided in PCT International Publication Number WO 2010/045599A1, published 22 April 2010. Compound 106, Compound 109, and Compound 110 are commercially available, e.g., from Sigma-Aldrich, St. Louis, MO, USA.

In certain embodiments, the trypsin inhibitor is SBTI, BBSI, Compound 101, Compound 106, Compound 108, Compound 109, or Compound 110. In certain embodiments, the trypsin inhibitor is camostat.

In certain embodiments, the trypsin inhibitor is a compound of formula T-I: 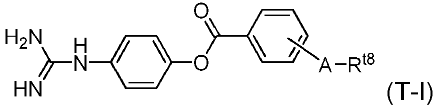 wherein
A represents a group of the following formula: 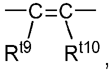
R^{t9} and R^{t10} each represents independently a hydrogen atom or a C₁₋₄ alkyl group,
R^{t8} represents a group selected from the following formulae: 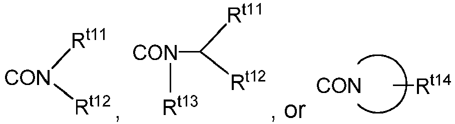
wherein R^{t11}, R^{t12} and R^{t13} each represents independently
   (1) a hydrogen atom,
   (2) a phenyl group,
   (3) a C₁₋₄ alkyl group substituted by a phenyl group,
   (4) a C₁₋₁₀ alkyl group,
   (5) a C₁₋₁₀ alkoxyl group,
   (6) a C₂₋₁₀ alkenyl group having 1 to 3 double bonds,
   (7) a C₂₋₁₀ alkynyl group having 1 to 2 triple bonds,
   (8) a group of formula: R^{t15}-C(O)XR^{t16},
wherein R^{t15} represents a single bond or a C₁₋₈ alkylene group,
X represents an oxygen atom or an NH-group, and
R^{t16} represents a hydrogen atom, a C₁₋₄ alkyl group, a phenyl group or a C₁₋₄ alkyl group substituted by a phenyl group, or
(9) a C₃₋₇ cycloalkyl group;
the structure 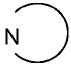 represents a 4-7 membered monocyclic hetero-ring containing 1 to 2 nitrogen or oxygen atoms,
R^{t14} represents a hydrogen atom, a C₁₋₄ alkyl group substituted by a phenyl group or a group of formula: COOR^{t17}, wherein R^{t17}represents a hydrogen atom, a C₁₋₄ alkyl group or a C₁₋₄ alkyl group substituted by a phenyl group;
provided that R^{t11}, R^{t12} and R^{t13} do not represent simultaneously hydrogen atoms;
or nontoxic salts, acid addition salts or hydrates thereof.

In certain embodiments, the trypsin inhibitor is a compound selected from the following: 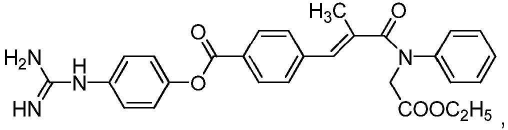 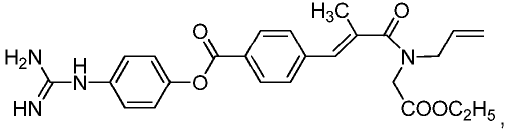 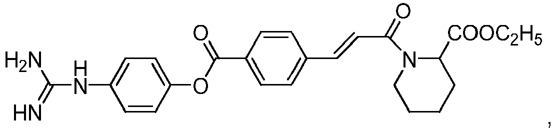 or

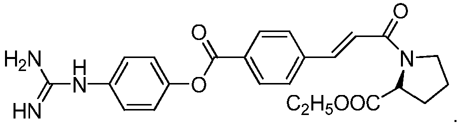

In certain embodiments, the trypsin inhibitor is a compound of formula T-II: 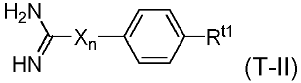 wherein
X is NH;
n is zero or one; and
R^{t1} is selected from hydrogen, halogen, nitro, alkyl, substituted alkyl, alkoxy, carboxyl, alkoxycarbonyl, acyl, aminoacyl, guanidine, amidino, carbamide, amino, substituted amino, hydroxyl, cyano and -(CH₂)ₘ-C(O)-O-(CH₂)ₘ-C(O)-N-Rⁿ¹Rⁿ², wherein each m is independently zero to 2; and Rⁿ¹ and Rⁿ², are independently selected from hydrogen and C₁₋₄ alkyl.

In certain embodiments, in formula T-II, R^{t1} is guanidino or amidino.

In certain embodiments, in formula T-II, R^{t1} is -(CH₂)ₘ-C(O)-O-(CH₂)ₘ-C(O)-N-Rⁿ¹Rⁿ², wherein m is one and Rⁿ¹ and Rⁿ², are methyl.

In certain embodiments, the trypsin inhibitor is a compound of formula T-III: 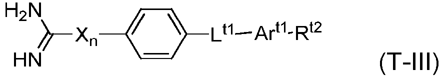 wherein
X is NH;
n is zero or one;
L^{t1} is selected from -C(O)-O- ; -O-C(O)-; -O-(CH₂)ₘ-O-;-OCH₂-Ar^{t2}-CH₂O-; - C(O)-NR^{t3}-; and - NR^{t3}-C(O)-;
R^{t3} is selected from hydrogen, C₁₋₆ alkyl, and substituted C₁₋₆ alkyl;
Ar^{t1} and Ar^{t2} are independently a substituted or unsubstituted aryl group;
m is a number from 1 to 3; and
R^{t2} is selected from hydrogen, halogen, nitro, alkyl, substituted alkyl, alkoxy, carboxyl, alkoxycarbonyl, acyl, aminoacyl, guanidine, amidino, carbamide, amino, substituted amino, hydroxyl, cyano and -(CH₂)ₘ-C(O)-O-(CH₂)ₘ-C(O)-N-Rⁿ¹Rⁿ², wherein each m is independently zero to 2; and Rⁿ¹ and Rⁿ² are independently selected from hydrogen and C₁₋₄ alkyl.

In certain embodiments, in formula T-III, R^{t2} is guanidino or amidino.

In certain embodiments, in formula T-III, R^{t2} is -(CH₂)ₘ-C(O)-O-(CH₂)ₘ-C(O)-N-Rⁿ¹Rⁿ², wherein m is one and Rⁿ¹ and Rⁿ² are methyl.

In certain embodiments, the trypsin inhibitor is a compound of formula T-IV: 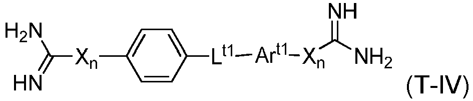 wherein
each X is NH;
each n is independently zero or one;
L^{t1} is selected from -C(O)-O- ; -O-C(O)-; -O-(CH₂)ₘ-O-;-OCH₂-Ar^{t2}-CH₂O-; - C(O)-NR^{t3}-; and - NR^{t3}-C(O)-;
R^{t3} is selected from hydrogen, C₁₋₆ alkyl, and substituted C₁₋₆ alkyl;
Ar^{t1} and Ar^{t2} are independently a substituted or unsubstituted aryl group; and
m is a number from 1 to 3.

In certain embodiments, in formula T-IV, Ar^{t1} or Ar^{t2} is phenyl.

In certain embodiments, in formula T-IV, Ar^{t1} or Ar^{t2} is naphthyl.

In certain embodiments, the trypsin inhibitor is Compound 109.

In certain embodiments, the trypsin inhibitor is

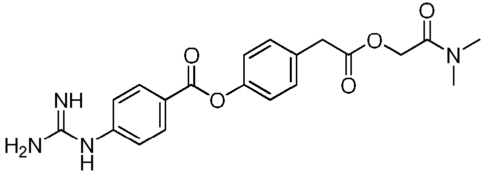

In certain embodiments, the trypsin inhibitor is Compound 110 or a bis-arylamidine variant thereof; see, for example, J.D. Geratz, M.C.-F. Cheng and R.R. Tidwell (1976) J Med. Chem. 19, 634-639.

It is to be appreciated that the invention also includes inhibitors of other enzymes involved in protein assimilation that can be used in combination with Compound AM-9 and Compound AM-10 to attenuate release of amphetamine from the prodrug.

### Combinations of Prodrug and Trypsin Inhibitor

As discussed above, the present disclosure provides pharmaceutical compositions which comprise a trypsin inhibitor and one or more of Compound AM-9 and Compound AM-10, an amphetamine prodrug, that comprises a promoiety comprising a trypsin-cleavable moiety that, when cleaved, facilitates release of amphetamine. Examples of compositions containing one or more of Compound AM-9 and Compound AM-10 and a trypsin inhibitor are described below.

The embodiments provide a pharmaceutical composition, which comprises a compound of Formulae T-I to T-IV and Compound AM-9, or a pharmaceutically acceptable salt thereof. The embodiments provide a pharmaceutical composition, which comprises Compound 109 and Compound AM-9, or a pharmaceutically acceptable salt thereof.

The embodiments provide a pharmaceutical composition, which comprises a compound of Formulae T-I to T-IV and Compound AM-10, or a pharmaceutically acceptable salt thereof. The embodiments provide a pharmaceutical composition, which comprises Compound 109 and Compound AM-10, or a pharmaceutically acceptable salt thereof.

Certain embodiments provide for a combination of one or more of Compound AM-9 and Compound AM-10 and a trypsin inhibitor, in which the trypsin inhibitor is shown in the following table.

| Prodrug | Trypsin inhibitor |
|---|---|
| Compound AM-9 | SBTI |
| Compound AM-9 | BBSI |
| Compound AM-9 | Compound 101 |
| Compound AM-9 | Compound 106 |
| Compound AM-9 | Compound 108 |
| Compound AM-9 | Compound 109 |
| Compound AM-9 | Compound 110 |
| Compound AM-9 | camostat |
| Compound AM-10 | SBTI |
| Compound AM-10 | BBSI |
| Compound AM-10 | Compound 101 |
| Compound AM-10 | Compound 106 |
| Compound AM-10 | Compound 108 |
| Compound AM-10 | Compound 109 |
| Compound AM-10 | Compound 110 |
| Compound AM-10 | camostat |

### Combinations of Amphetamine Prodrugs and Other Drugs

The disclosure provides for an amphetamine prodrug and a further prodrug or drug included in a pharmaceutical composition. Such a prodrug or drug may provide additional stimulant effects or may have effects other than, or in addition to, the effects associated with amphetamines. Embodiments provide a pharmaceutical composition, which comprises an amphetamine prodrug and a further prodrug or drug and optionally comprises an enzyme inhibitor. Also included are pharmaceutically acceptable salts thereof.

In certain embodiments, the enzyme inhibitor is selected from SBTI, BBSI, Compound 101, Compound 106, Compound 108, Compound 109, and Compound 110. In certain embodiments, the enzyme inhibitor is camostat.

In certain embodiments, a pharmaceutical composition can comprise an amphetamine prodrug, a non-amphetamine drug and at least one enzyme inhibitor.

### Pharmaceutical Compositions and Methods of Use

The pharmaceutical composition according to the embodiments can further comprise a pharmaceutically acceptable carrier. The composition is conveniently formulated in a form suitable for oral (including buccal and sublingual) administration, for example as a tablet, capsule, thin film, powder, suspension, solution, syrup, dispersion or emulsion. The composition can contain components conventional in pharmaceutical preparations, e.g., one or more carriers, binders, lubricants, excipients (e.g., to impart controlled release characteristics), pH modifiers, sweeteners, bulking agents, coloring agents or further active agents.

Patients can be humans, and also other mammals, such as livestock, zoo animals and companion animals, such as a cat, dog or horse.

In some aspects, the embodiments provide a pharmaceutical composition as described herein for use in the treatment of conditions such as, but not limited to, Attention Deficit Hyperactivity Disorder (ADHD), Chronic Fatigue Syndrome (CFS), brain injuries, narcolepsy, obesity, etc. The pharmaceutical composition according to the embodiments is useful, for example, in the treatment of a patient suffering from, ADHD, CFS, brain injury, narcolepsy, obesity, etc. Accordingly, described herein are methods of treating or preventing ADHD, CFS, brain injury, narcolepsy, or obesity in a subject, the methods involving administering to the subject a disclosed composition. The present disclosure provides for a disclosed composition for use in therapy or prevention or as a medicament. The present disclosure also provides the use of a disclosed composition for the manufacture of a medicament, especially for the manufacture of a medicament for the treatment or prevention of ADHD, CFS, brain injury, narcolepsy, or obesity.

Described herein is the use of an amphetamine prodrug (Compound AM-9, Compound AM-10) and an enzyme inhibitor, such as a trypsin inhibitor, in the treatment of ADHD, CFS, brain injury, narcolepsy, or obesity. Described herein is the use of an amphetamine prodrug and an enzyme inhibitor, such as a trypsin inhibitor, in the prevention of ADHD, CFS, brain injury, narcolepsy, or obesity.

The present disclosure provides use of an amphetamine prodrug (Compound AM-9, Compound AM-10) and an enzyme inhibitor, such as a trypsin inhibitor, in the manufacture of a medicament for treatment of ADHD, CFS, brain injury, narcolepsy, or obesity. The present disclosure provides use of an amphetamine prodrug and an enzyme inhibitor, such as a trypsin inhibitor, in the manufacture of a medicament for prevention of ADHD, CFS, brain injury, narcolepsy, or obesity.

Described herein is a method of treating ADHD, CFS, brain injury, narcolepsy, or obesity in a patient requiring treatment, which comprises administering an effective amount of a pharmaceutical composition as described herein. Described herein is a method of preventing ADHD, CFS, brain injury, narcolepsy, or obesity in a patient requiring treatment, which comprises administering an effective amount of a pharmaceutical composition as described herein.

The amount of composition disclosed herein to be administered to a patient to be effective (i.e., to provide blood levels of amphetamine sufficient to be effective in the treatment or prophylaxis of ADHD, CFS, brain injury, narcolepsy, or obesity) will depend upon the bioavailability of the particular composition, the susceptibility of the particular composition to enzyme activation in the gut, the amount and potency of enzyme inhibitor (e.g., trypsin inhibitor) present in the composition, as well as other factors, such as the species, age, weight, sex, and condition of the patient, manner of administration and judgment of the prescribing physician. In general, the composition dose can be such that the amphetamine prodrug is in the range of from 0.01 milligrams prodrug per kilogram to 20 milligrams prodrug per kilogram (mg/kg) body weight. For example, a composition comprising a residue of amphetamine can be administered at a dose equivalent to administering free amphetamine in the range of from 0.01 mg/kg to 40 mg/kg body weight, or 0.1 to 30 mg/kg body weight, or 0.2 to 20 mg/kg body weight. In one embodiment wherein the composition comprises an amphetamine prodrug, the composition can be administered at a dose such that the level of amphetamine achieved in the blood is in the range of from 0.5 ng/ml to 200 ng/ml.

The amount of an enzyme inhibitor (e.g., a trypsin inhibitor) to be administered to the patient to be effective (i.e., to attenuate release of amphetamine when administration of an amphetamine prodrug disclosed herein alone would lead to overexposure of amphetamine) will depend upon the effective dose of the particular prodrug and the potency of the particular enzyme inhibitor, as well as other factors, such as the species, age, weight, sex and condition of the patient, manner of administration and judgment of the prescribing physician. In general, the dose of enzyme inhibitor can be in the range of from 0.001 mg to 50 mg per mg of prodrug disclosed herein. In a certain embodiment, the dose of enzyme inhibitor can be in the range of from 0.05 mg to 50 mg per mg of prodrug disclosed herein. In one embodiment, the dose of enzyme inhibitor can be in the range of from 0.01 nanomoles to 100 micromoles per micromole of prodrug (Compound AM-9, Compound AM-10) disclosed herein.

### Representative Embodiments of Dose Units of Prodrug Compound AM-9 and Trypsin Inhibitor Having a Desired Pharmacokinetic Profile

The embodiments include a composition that comprises (a) a prodrug comprising amphetamine covalently bound to a promoiety comprising a trypsin-cleavable moiety, wherein cleavage of the trypsin-cleavable moiety by trypsin mediates release of amphetamine, wherein the prodrug is Compound AM-9 and (b) a trypsin inhibitor that interacts with the trypsin that mediates enzymatically-controlled release of amphetamine from the prodrug following ingestion of the composition.

The embodiments include a dose unit comprising a composition, such as a pharmaceutical composition, comprising Compound AM-9, an amphetamine-modified prodrug, and a trypsin inhibitor, where Compound AM-9 and trypsin inhibitor are present in the dose unit in an amount effective to provide for a pre-selected pharmacokinetic (PK) profile following ingestion. In further embodiments, the pre-selected PK profile comprises at least one PK parameter value that is less than the PK parameter value of amphetamine released following ingestion of an equivalent dosage of Compound AM-9 in the absence of inhibitor. In further embodiments, the PK parameter value is selected from an amphetamine Cmax value, an amphetmine exposure value, and a (1/ amphetamine Tmax) value.

In certain embodiments, the dose unit provides for a pre-selected PK profile following ingestion of at least two dose units. In related embodiments, the pre-selected PK profile of such dose units is modified relative to the PK profile following ingestion of an equivalent dosage of Compound AM-9 without inhibitor. In related embodiments, such a dose unit provides that ingestion of an increasing number of the dose units provides for a linear PK profile. In related embodiments, such a dose unit provides that ingestion of an increasing number of the dose units provides for a nonlinear PK profile. In related embodiments, the PK parameter value of the PK profile of such a dose unit is selected from an amphetamine Cmax value, a (1/ amphetamine Tmax) value, and an amphetamine exposure value.

Described herein are methods for treating a patient comprising administering any of the compositions, such as pharmaceutical compositions, comprising Compound AM-9 and a trypsin inhibitor or dose units described herein to a patient in need thereof. Described herein are methods to reduce side effects of a therapy comprising administering any of such compositions, e.g., pharmaceutical compositions, or dose units described herein to a patient in need thereof. Described herein are methods of improving patient compliance with a therapy prescribed by a clinician comprising directing administration of any of such compositions, e.g., pharmaceutical compositions, or dose units described herein to a patient in need thereof. Such methods can provide for improved patient compliance with a prescribed therapy as compared to patient compliance with a prescribed therapy using drug and/or using prodrug without inhibitor as compared to prodrug with inhibitor.

Also described herein are methods of reducing risk of unintended overdose of amphetamine comprising directing administration of any of such compositions, e.g., pharmaceutical compositions, or dose units described herein to a patient in need of treatment.

Also described herein are methods of making a dose unit comprising combining Compound AM-9 and a trypsin inhibitor in a dose unit, wherein Compound AM-9 and trypsin inhibitor are present in the dose unit in an amount effective to attenuate release of amphetamine from Compound AM-9.

Also described herein are methods of deterring misuse or abuse of multiple dose units of Compound AM-9 comprising combining Compound AM-9 and a trypsin inhibitor in a dose unit, wherein Compound AM-9 and trypsin inhibitor are present in the dose unit in an amount effective to attenuate release of amphetamine from Compound AM-9 such that ingestion of multiples of dose units by a patient does not provide a proportional release of amphetamine. In further embodiments, release of drug is decreased compared to release of drug by an equivalent dosage of prodrug in the absence of inhibitor.

One embodiment is a method for identifying a trypsin inhibitor and prodrug Compound AM-9 suitable for formulation in a dose unit. Such a method can be conducted as, for example, an *in vitro* assay or an *ex vivo* assay.

The embodiments include methods for identifying a trypsin inhibitor and prodrug Compound AM-9 suitable for formulation in a dose unit comprising combining prodrug Compound AM-9, a trypsin inhibitor, and trypsin in a reaction mixture, and detecting prodrug conversion, wherein a decrease in prodrug conversion in the presence of the trypsin inhibitor as compared to prodrug conversion in the absence of the trypsin inhibitor indicates the trypsin inhibitor and prodrug Compound AM-9 are suitable for formulation in a dose unit.

Also described herein are methods for identifying a trypsin inhibitor and prodrug Compound AM-9 suitable for formulation in a dose unit comprising administering to an animal a trypsin inhibitor and prodrug Compound AM-9 and detecting prodrug conversion, wherein a decrease in ampheatmine conversion in the presence of the trypsin inhibitor as compared to amphetamine conversion in the absence of the trypsin inhibitor indicates the trypsin inhibitor and prodrug Compound AM-9 are suitable for formulation in a dose unit. The administering may comprise administering to the animal increasing doses of inhibitor co-dosed with a selected fixed dose of prodrug. Detecting prodrug conversion can facilitate identification of a dose of inhibitor and a dose of prodrug that provides for a pre-selected pharmacokinetic (PK) profile. Such methods can be conducted as, for example, an *in vivo* assay or an *ex vivo* assay.

The embodiments include methods for identifying a trypsin inhibitor and prodrug Compound AM-9 suitable for formulation in a dose unit comprising administering to an animal tissue a trypsin inhibitor and prodrug Compound AM-9 and detecting prodrug conversion, wherein a decrease in prodrug conversion in the presence of the trypsin inhibitor as compared to prodrug conversion in the absence of the trypsin inhibitor indicates the trypsin inhibitor and prodrug Compound AM-9 are suitable for formulation in a dose unit.

### Representative Embodiments of Dose Units of Prodrug Compound AM-10 and Trypsin Inhibitor Having a Desired Pharmacokinetic Profile

The embodiments include a composition that comprises (a) a prodrug comprising amphetamine covalently bound to a promoiety comprising a trypsin-cleavable moiety, wherein cleavage of the trypsin-cleavable moiety by trypsin mediates release of amphetamine, wherein the prodrug is Compound AM-10 and (b) a trypsin inhibitor that interacts with the trypsin that mediates enzymatically-controlled release of amphetamine from the prodrug following ingestion of the composition.

The embodiments include a dose unit comprising a composition, such as a pharmaceutical composition, comprising Compound AM-10, an amphetamine-modified prodrug, and a trypsin inhibitor, where Compound AM-10 and trypsin inhibitor are present in the dose unit in an amount effective to provide for a pre-selected pharmacokinetic (PK) profile following ingestion. In further embodiments, the pre-selected PK profile comprises at least one PK parameter value that is less than the PK parameter value of amphetamine released following ingestion of an equivalent dosage of Compound AM-10 in the absence of inhibitor. In further embodiments, the PK parameter value is selected from an amphetamine Cmax value, an amphetmine exposure value, and a (1/ amphetamine Tmax) value.

In certain embodiments, the dose unit provides for a pre-selected PK profile following ingestion of at least two dose units. In related embodiments, the pre-selected PK profile of such dose units is modified relative to the PK profile following ingestion of an equivalent dosage of Compound AM-10 without inhibitor. In related embodiments, such a dose unit provides that ingestion of an increasing number of the dose units provides for a linear PK profile. In related embodiments, such a dose unit provides that ingestion of an increasing number of the dose units provides for a nonlinear PK profile. In related embodiments, the PK parameter value of the PK profile of such a dose unit is selected from an amphetamine Cmax value, a (1/ amphetamine Tmax) value, and an amphetamine exposure value.

Described herein are methods for treating a patient comprising administering any of the compositions, such as pharmaceutical compositions, comprising Compound AM-10 and a trypsin inhibitor or dose units described herein to a patient in need thereof. Described herein are methods to reduce side effects of a therapy comprising administering any of such compositions, e.g., pharmaceutical compositions, or dose units described herein to a patient in need thereof. Also described herein are methods of improving patient compliance with a therapy prescribed by a clinician comprising directing administration of any of such compositions, e.g., pharmaceutical compositions, or dose units described herein to a patient in need thereof. Such methods can provide for improved patient compliance with a prescribed therapy as compared to patient compliance with a prescribed therapy using drug and/or using prodrug without inhibitor as compared to prodrug with inhibitor.

Also described herein are methods of reducing risk of unintended overdose of amphetamine comprising directing administration of any of such compositions, e.g., pharmaceutical compositions, or dose units described herein to a patient in need of treatment.

The embodiments include methods of making a dose unit comprising combining Compound AM-10 and a trypsin inhibitor in a dose unit, wherein Compound AM-10 and trypsin inhibitor are present in the dose unit in an amount effective to attenuate release of amphetamine from Compound AM-10.

The embodiments include methods of deterring misuse or abuse of multiple dose units of Compound AM-10 comprising combining Compound AM-10 and a trypsin inhibitor in a dose unit, wherein Compound AM-10 and trypsin inhibitor are present in the dose unit in an amount effective to attenuate release of amphetamine from Compound AM-10 such that ingestion of multiples of dose units by a patient does not provide a proportional release of amphetamine. In further embodiments, release of drug is decreased compared to release of drug by an equivalent dosage of prodrug in the absence of inhibitor.

One embodiment is a method for identifying a trypsin inhibitor and prodrug Compound AM-10 suitable for formulation in a dose unit. Such a method can be conducted as, for example, an *in vitro* assay or an *ex vivo* assay.

The embodiments include methods for identifying a trypsin inhibitor and prodrug Compound AM-10 suitable for formulation in a dose unit comprising combining prodrug Compound AM-10, a trypsin inhibitor, and trypsin in a reaction mixture, and detecting prodrug conversion, wherein a decrease in prodrug conversion in the presence of the trypsin inhibitor as compared to prodrug conversion in the absence of the trypsin inhibitor indicates the trypsin inhibitor and prodrug Compound AM-10 are suitable for formulation in a dose unit.

Also described herein are methods for identifying a trypsin inhibitor and prodrug Compound AM-10 suitable for formulation in a dose unit comprising administering to an animal a trypsin inhibitor and prodrug Compound AM-10 and detecting prodrug conversion, wherein a decrease in ampheatmine conversion in the presence of the trypsin inhibitor as compared to amphetamine conversion in the absence of the trypsin inhibitor indicates the trypsin inhibitor and prodrug Compound AM-10 are suitable for formulation in a dose unit. The administering may comprise administering to the animal increasing doses of inhibitor co-dosed with a selected fixed dose of prodrug. Detecting prodrug conversion can facilitate identification of a dose of inhibitor and a dose of prodrug that provides for a pre-selected pharmacokinetic (PK) profile. Such methods can be conducted as, for example, an *in vivo* assay or an *ex vivo* assay.

The embodiments include methods for identifying a trypsin inhibitor and prodrug Compound AM-10 suitable for formulation in a dose unit comprising administering to an animal tissue a trypsin inhibitor and prodrug Compound AM-10 and detecting prodrug conversion, wherein a decrease in prodrug conversion in the presence of the trypsin inhibitor as compared to prodrug conversion in the absence of the trypsin inhibitor indicates the trypsin inhibitor and prodrug Compound AM-10 are suitable for formulation in a dose unit.

### Dose Units of Prodrug Compound AM-9 or Compound AM-10 and Trypsin Inhibitor Having a Desired Pharmacokinetic Profile

The present disclosure provides dose units of prodrug and inhibitor that can provide for a desired pharmacokinetic (PK) profile. Dose units can provide a modified PK profile compared to a reference PK profile as disclosed herein. It will be appreciated that a modified PK profile can provide for a modified pharmacodynamic (PD) profile. Ingestion of multiples of such a dose unit can also provide a desired PK profile.

Unless specifically stated otherwise, "dose unit" as used herein refers to a combination of a trypsin-cleavable prodrug and a trypsin inhibitor. A "single dose unit" is a single unit of a combination of a trypsin-cleavable prodrug and a trypsin inhibitor, where the single dose unit provide a therapeutically effective amount of drug (i.e., a sufficient amount of drug to effect a therapeutic effect, e.g., a dose within the respective drug's therapeutic window, or therapeutic range). "Multiple dose units" or "multiples of a dose unit" or a "multiple of a dose unit" refers to at least two single dose units.

As used herein, a "PK profile" refers to a profile of drug concentration in blood or plasma. Such a profile can be a relationship of drug concentration over time (i.e., a "concentration-time PK profile") or a relationship of drug concentration versus number of doses ingested (i.e., a "concentration-dose PK profile".) A PK profile is characterized by PK parameters.

As used herein, a "PK parameter" refers to a measure of drug concentration in blood or plasma, such as: 1) "drug Cmax", the maximum concentration of drug achieved in blood or plasma; 2) "drug Tmax", the time elapsed following ingestion to achieve Cmax; and 3) "drug exposure", the total concentration of drug present in blood or plasma over a selected period of time, which can be measured using the area under the curve (AUC) of a time course of drug release over a selected period of time (t). Modification of one or more PK parameters provides for a modified PK profile.

For purposes of describing the features of dose units of the present disclosure, "PK parameter values" that define a PK profile include drug Cmax (e.g., amphetamine Cmax), total drug exposure (e.g., area under the curve) (e.g., amphetamine exposure) and 1/(drug Tmax) (such that a decreased 1/Tmax is indicative of a delay in Tmax relative to a reference Tmax) (e.g., 1/amphetamine Tmax). Thus a decrease in a PK parameter value relative to a reference PK parameter value can indicate, for example, a decrease in drug Cmax, a decrease in drug exposure, and/or a delayed Tmax.

Dose units of the present disclosure can be adapted to provide for a modified PK profile, e.g., a PK profile that is different from that achieved from dosing a given dose of prodrug in the absence of inhibitor (i.e., without inhibitor). For example, dose units can provide for at least one of decreased drug Cmax, delayed drug Tmax and/or decreased drug exposure compared to ingestion of a dose of prodrug in the same amount but in the absence of inhibitor. Such a modification is due to the inclusion of an inhibitor in the dose unit.

As used herein, "a pharmacodynamic (PD) profile" refers to a profile of the efficacy of a drug in a patient (or subject or user), which is characterized by PD parameters. "PD parameters" include "drug Emax" (the maximum drug efficacy), "drug EC50" (the concentration of drug at 50% of the Emax), and side effects.

At low concentrations of inhibitor, there may be no detectable effect on drug release, as illustrated by the plateau portion of the plot of drug Cmax (Y axis) versus inhibitor concentration (X axis). As inhibitor concentration increases, a concentration is reached at which drug release from prodrug is attenuated, causing a decrease in, or suppression of, drug Cmax. Thus, the effect of inhibitor upon a prodrug PK parameter for a dose unit of the present disclosure can range from undetectable, to moderate, to complete inhibition (i.e., no detectable drug release).

A dose unit can be adapted to provide for a desired PK profile (e.g., a concentration-time PK profile) following ingestion of a single dose. A dose unit can be adapted to provide for a desired PK profile (e.g., a concentration-dose PK profile) following ingestion of multiple dose units (e.g., at least 2, at least 3, at least 4 or more dose units).

### Dose units providing modified PK profiles

A combination of a prodrug and an inhibitor in a dose unit can provide a desired (or "pre-selected") PK profile (e.g., a concentration-time PK profile) following ingestion of a single dose. The PK profile of such a dose unit can be characterized by one or more of a pre-selected drug Cmax, a pre-selected drug Tmax or a pre-selected drug exposure. The PK profile of the dose unit can be modified compared to a PK profile achieved from the equivalent dosage of prodrug in the absence of inhibitor (i.e., a dose that is the same as the dose unit except that it lacks inhibitor).

A modified PK profile can have a decreased PK parameter value relative to a reference PK parameter value (e.g., a PK parameter value of a PK profile following ingestion of a dosage of prodrug that is equivalent to a dose unit except without inhibitor). For example, a dose unit can provide for a decreased drug Cmax, decreased drug exposure, and/or delayed drug Tmax.

Dose units that provide for a modified PK profile (e.g., a decreased drug Cmax and/or delayed drug Tmax as compared to, a PK profile of drug or a PK profile of prodrug without inhibitor), find use in tailoring of drug dose according to a patient's needs (e.g., through selection of a particular dose unit and/or selection of a dosage regimen), reduction of side effects, and/or improvement in patient compliance (as compared to side effects or patient compliance associated with drug or with prodrug without inhibitor). As used herein, "patient compliance" refers to whether a patient follows the direction of a clinician (e.g., a physician) including ingestion of a dose that is neither significantly above nor significantly below that prescribed. Such dose units also reduce the risk of misuse, abuse or overdose by a patient as compared to such risk(s) associated with drug or prodrug without inhibitor. For example, dose units with a decreased drug Cmax provide less reward for ingestion than does a dose of the same amount of drug, and/or the same amount of prodrug without inhibitor.

### Dose units providing modified PK profiles upon ingestion of multiple dose units

A dose unit of the present disclosure can be adapted to provide for a desired PK profile (e.g., a concentration-time PK profile or concentration-dose PK profile) following ingestion of multiples of a dose unit (e.g., at least 2, at least 3, at least 4, or more dose units). A concentration-dose PK profile refers to the relationship between a selected PK parameter and a number of single dose units ingested. Such a profile can be dose proportional, linear (a linear PK profile) or nonlinear (a nonlinear PK profile). A modified concentration-dose PK profile can be provided by adjusting the relative amounts of prodrug and inhibitor contained in a single dose unit and/or by using a different prodrug and/or inhibitor.

It is to be appreciated that a concentration-dose PK profile resulting from ingestion of multiples of a dose unit of the disclosure can also be compared to other references, such as a concentration-dose PK profile provided by ingestion of an increasing number of doses of prodrug without inhibitor wherein the amount of drug released into the blood or plasma by a single dose of prodrug in the absence of inhibitor represents a therapeutically effective amount equivalent to the amount of drug released into the blood or plasma by one dose unit of the disclosure.

A dose unit can include inhibitor in an amount that does not detectably affect drug release following ingestion. Ingestion of multiples of such a dose unit can provide a concentration-dose PK profile such that the relationship between number of dose units ingested and PK parameter value is linear with a positive slope, which is similar to, for example, a dose proportional PK profile of increasing amounts of prodrug alone. Dose units that provide a concentration-dose PK profile having such an undetectable change in drug Cmax in vivo compared to the profile of prodrug alone can find use in thwarting enzyme conversion of prodrug from a dose unit that has sufficient inhibitor to reduce or prevent in vitro cleavage of the enzyme-cleavable prodrug by its respective enzyme.

Concentration-dose PK profiles following ingestion of multiples of a dose unit can be linear with positive slope, where the profile exhibits a reduced slope. Such a dose unit provides a profile having a decreased PK parameter value (e.g., drug Cmax) relative to a reference PK parameter value exhibiting dose proportionality.

Concentration-dose PK profiles following ingestion of multiples of a dose unit can be non-linear. In a non-linear, the biphasic concentration-dose PK profile contains a first phase over which the concentration-dose PK profile has a positive rise, and then a second phase over which the relationship between number of dose units ingested and a PK parameter value (e.g., drug Cmax) is relatively flat (substantially linear with zero slope). For such a dose unit, for example, drug Cmax can be increased for a selected number of dose units (e.g., 2, 3, or 4 dose units). However, ingestion of additional dose units does not provide for a significant increase in drug Cmax.

In addition, the biphasic concentration-dose PK profile is characterized by a first phase over which the concentration-dose PK profile has a positive rise and a second phase over which the relationship between number of dose units ingested and a PK parameter value (e.g., drug Cmax) declines. Dose units that provide this concentration-dose PK profile provide for an increase in drug Cmax for a selected number of ingested dose units (e.g., 2, 3, or 4 dose units). However, ingestion of further additional dose units does not provide for a significant increase in drug Cmax and instead provides for decreased drug Cmax.

A concentration-dose PK profile in which the relationship between the number of dose units ingested and a PK parameter (e.g., drug Cmax) is linear with zero slope do not provide for a significant increase or decrease in drug Cmax with ingestion of multiples of dose units.

A concentration-dose PK profile in which the relationship between number of dose units ingested and a PK parameter value (e.g., drug Cmax) is linear with a negative slope. Thus drug Cmax decreases as the number of dose units ingested increases.

Dose units that provide for concentration-dose PK profiles when multiples of a dose unit are ingested find use in tailoring of a dosage regimen to provide a therapeutic level of released drug while reducing the risk of overdose, misuse, or abuse. Such reduction in risk can be compared to a reference, e.g., to administration of drug alone or prodrug alone. In one embodiment, risk is reduced compared to administration of a drug or prodrug that provides a proportional concentration-dose PK profile. A dose unit that provides for a concentration-dose PK profile can reduce the risk of patient overdose through inadvertent ingestion of dose units above a prescribed dosage. Such a dose unit can reduce the risk of patient misuse (e.g., through self-medication). Such a dose unit can discourage abuse through deliberate ingestion of multiple dose units. For example, a dose unit that provides for a biphasic concentration-dose PK profile can allow for an increase in drug release for a limited number of dose units ingested, after which an increase in drug release with ingestion of more dose units is not realized. In another example, a dose unit that provides for a concentration-dose PK profile of zero slope can allow for retention of a similar drug release profile regardless of the number of dose units ingested.

Ingestion of multiples of a dose unit can provide for adjustment of a PK parameter value relative to that of ingestion of multiples of the same dose (either as drug alone or as a prodrug) in the absence of inhibitor such that, for example, ingestion of a selected number (e.g., 2, 3, 4 or more) of a single dose unit provides for a decrease in a PK parameter value compared to ingestion of the same number of doses in the absence of inhibitor.

Pharmaceutical compositions include those having an inhibitor to provide for protection of a therapeutic compound from degradation in the GI tract. Inhibitor can be combined with a drug (i.e., not a prodrug) to provide for protection of the drug from degradation in the GI system. In this example, the composition of inhibitor and drug provide for a modified PK profile by increasing a PK parameter. Inhibitor can also be combined with a prodrug that is susceptible to degradation by a GI enzyme and has a site of action outside the GI tract. In this composition, the inhibitor protects ingested prodrug in the GI tract prior to its distribution outside the GI tract and cleavage at a desired site of action.

### Methods used to define relative amounts of prodrug and inhibitor in a dose unit

Dose units that provide for a desired PK profile, such as a desired concentration-time PK profile and/or a desired concentration-dose PK profile, can be made by combining a prodrug and an inhibitor in a dose unit in relative amounts effective to provide for release of drug that provides for a desired drug PK profile following ingestion by a patient.

Prodrugs can be selected as suitable for use in a dose unit by determining the trypsin-mediated drug release competency of the prodrug. This can be accomplished in vitro, in vivo or ex vivo.

In vitro assays can be conducted by combining a prodrug with trypsin in a reaction mixture. Trypsin can be provided in the reaction mixture in an amount sufficient to catalyze cleavage of the prodrug. Assays are conducted under suitable conditions, and optionally may be under conditions that mimic those found in a GI tract of a subject, e.g., human. "Prodrug conversion" refers to release of drug from prodrug. Prodrug conversion can be assessed by detecting a level of a product of prodrug conversion (e.g., released drug) and/or by detecting a level of prodrug that is maintained in the presence of trypsin. Prodrug conversion can also be assessed by detecting the rate at which a product of prodrug conversion occurs or the rate at which prodrug disappears. An increase in released drug, or a decrease in prodrug, indicate prodrug conversion has occurred. Prodrugs that exhibit an acceptable level of prodrug conversion in the presence of trypsin within an acceptable period of time are suitable for use in a dose unit in combination with a trypsin inhibitor.

In vivo assays can assess the suitability of a prodrug for use in a dose unit by administration of the prodrug to an animal (e.g., a human or non-human animal, e.g., rat, dog, pig, etc.). Such administration can be enteral (e.g., oral administration). Prodrug conversion can be detected by, for example, detecting a product of prodrug conversion (e.g., released drug or a metabolite of released drug) or detecting prodrug in blood or plasma of the animal at a desired time point(s) following administration.

Ex vivo assays, such as a gut loop or inverted gut loop assay, can assess the suitability of a prodrug for use in a dose unit by, for example, administration of the prodrug to a ligated section of the intestine of an animal. Prodrug conversion can be detected by, for example, detecting a product of prodrug conversion (e.g., released drug or a metabolite of released drug) or detecting prodrug in the ligated gut loop of the animal at a desired time point(s) following administration.

Inhibitors are generally selected based on, for example, activity in interacting with trypsin that mediates release of drug from a prodrug with which the inhibitor is to be co-dosed. Such assays can be conducted in the presence of enzyme either with or without prodrug. Inhibitors can also be selected according to properties such as half-life in the GI system, potency, avidity, affinity, molecular size and/or enzyme inhibition profile (e.g., steepness of inhibition curve in an enzyme activity assay, inhibition initiation rate). Inhibitors for use in prodrug-inhibitor combinations can be selected through use of in vitro, in vivo and/or ex vivo assays.

One embodiment is a method for identifying a prodrug and a trypsin inhibitor suitable for formulation in a dose unit wherein the method comprises combining a prodrug (e.g., Compound AM-9 or Compound AM-10), a trypsin inhibitor, and trypsin in a reaction mixture and detecting prodrug conversion. Such a combination is tested for an interaction between the prodrug, inhibitor and enzyme, i.e., tested to determine how the inhibitor will interact with the enzyme that mediates enzymatically-controlled release of the drug from the prodrug. In one embodiment, a decrease in prodrug conversion in the presence of the trypsin inhibitor as compared to prodrug conversion in the absence of the trypsin inhibitor indicates the prodrug and trypsin inhibitor are suitable for formulation in a dose unit. Such a method can be an in vitro assay.

Also described herein is a method for identifying a prodrug and a trypsin inhibitor suitable for formulation in a dose unit wherein the method comprises administering to an animal a prodrug (e.g., Compound AM-9 or Compound AM-10) and a trypsin inhibitor and detecting prodrug conversion. A decrease in prodrug conversion in the presence of the trypsin inhibitor as compared to prodrug conversion in the absence of the trypsin inhibitor may indicate the prodrug and trypsin inhibitor are suitable for formulation in a dose unit. Such a method can be an in vivo assay; for example, the prodrug and trypsin inhibitor can be administered orally. Such a method can also be an ex vivo assay; for example, the prodrug and trypsin inhibitor can be administered orally or to a tissue, such as an intestine, that is at least temporarily exposed. Detection can occur in the blood or plasma or respective tissue. As used herein, tissue refers to the tissue itself and can also refer to contents within the tissue.

One embodiment is a method for identifying a prodrug and a trypsin inhibitor suitable for formulation in a dose unit wherein the method comprises administering a prodrug and a trypsin inhibitor to an animal tissue that has removed from an animal and detecting prodrug conversion. In one embodiment, a decrease in prodrug conversion in the presence of the trypsin inhibitor as compared to prodrug conversion in the absence of the trypsin inhibitor indicates the prodrug and trypsin inhibitor are suitable for formulation in a dose unit.

In vitro assays can be conducted by combining a prodrug, a trypsin inhibitor and trypsin in a reaction mixture. Trypsin can be provided in the reaction mixture in an amount sufficient to catalyze cleavage of the prodrug, and assays conducted under suitable conditions, optionally under conditions that mimic those found in a GI tract of a subject, e.g., human. Prodrug conversion can be assessed by detecting a level of a product of prodrug conversion (e.g., released drug) and/or by detecting a level of prodrug maintained in the presence of trypsin. Prodrug conversion can also be assessed by detecting the rate at which a product of prodrug conversion occurs or the rate at which prodrug disappears. Prodrug conversion that is modified in the presence of inhibitor as compared to a level of prodrug conversion in the absence of inhibitor indicates the inhibitor is suitable for attenuation of prodrug conversion and for use in a dose unit. Reaction mixtures having a fixed amount of prodrug and increasing amounts of inhibitor, or a fixed amount of inhibitor and increasing amounts of prodrug, can be used to identify relative amounts of prodrug and inhibitor which provide for a desired modification of prodrug conversion.

In vivo assays can assess combinations of prodrugs and inhibitors by co-dosing of prodrug and inhibitor to an animal. Such co-dosing can be enteral. "Co-dosing" refers to administration of prodrug and inhibitor as separate doses or a combined dose (i.e., in the same formulation). Prodrug conversion can be detected by, for example, detecting a product of prodrug conversion (e.g., released drug or drug metabolite) or detecting prodrug in blood or plasma of the animal at a desired time point(s) following administration. Combinations of prodrug and inhibitor can be identified that provide for a prodrug conversion level that yields a desired PK profile as compared to, for example, prodrug without inhibitor.

Combinations of relative amounts of prodrug and inhibitor that provide for a desired PK profile can be identified by dosing animals with a fixed amount of prodrug and increasing amounts of inhibitor, or with a fixed amount of inhibitor and increasing amounts of prodrug. One or more PK parameters can then be assessed, e.g., drug Cmax, drug Tmax, and drug exposure. Relative amounts of prodrug and inhibitor that provide for a desired PK profile are identified as amounts of prodrug and inhibitor for use in a dose unit. The PK profile of the prodrug and inhibitor combination can be, for example, characterized by a decreased PK parameter value relative to prodrug without inhibitor. A decrease in the PK parameter value of an inhibitor-to-prodrug combination (e.g., a decrease in drug Cmax, a decrease in 1/drug Tmax (i.e., a delay in drug Tmax) or a decrease in drug exposure) relative to a corresponding PK parameter value following administration of prodrug without inhibitor can be indicative of an inhibitor-to-prodrug combination that can provide a desired PK profile. Assays can be conducted with different relative amounts of inhibitor and prodrug.

In vivo assays can be used to identify combinations of prodrug and inhibitor that provide for dose units that provide for a desired concentration-dose PK profile following ingestion of multiples of the dose unit (e.g., at least 2, at least 3, at least 4 or more). Ex vivo assays can be conducted by direct administration of prodrug and inhibitor into a tissue and/or its contents of an animal, such as the intestine, including by introduction by injection into the lumen of a ligated intestine (e.g., a gut loop, or intestinal loop, assay, or an inverted gut assay). An ex vivo assay can also be conducted by excising a tissue and/or its contents from an animal and introducing prodrug and inhibitor into such tissues and/or contents.

For example, a dose of prodrug that is desired for a single dose unit is selected (e.g., an amount that provides an efficacious plasma drug level). A multiple of single dose units for which a relationship between that multiple and a PK parameter to be tested is then selected. For example, if a concentration-dose PK profile is to be designed for ingestion of 2, 3, 4, 5, 6, 7, 8, 9 or 10 dose units, then the amount of prodrug equivalent to ingestion of that same number of dose units is determined (referred to as the "high dose"). The multiple of dose units can be selected based on the number of ingested pills at which drug Cmax is modified relative to ingestion of the single dose unit. If, for example, the profile is to provide for abuse deterrence, then a multiple of 10 can be selected, for example. A variety of different inhibitors (e.g., from a panel of inhibitors) can be tested using different relative amounts of inhibitor and prodrug. Assays can be used to identify suitable combination(s) of inhibitor and prodrug to obtain a single dose unit that is therapeutically effective, wherein such a combination, when ingested as a multiple of dose units, provides a modified PK parameter compared to ingestion of the same multiple of drug or prodrug alone (wherein a single dose of either drug or prodrug alone releases into blood or plasma the same amount of drug as is released by a single dose unit).

Increasing amounts of inhibitor are then co-dosed to animals with the high dose of prodrug. The dose level of inhibitor that provides a desired drug Cmax following ingestion of the high dose of prodrug is identified and the resultant inhibitor-to-prodrug ratio determined.

Prodrug and inhibitor are then co-dosed in amounts equivalent to the inhibitor-to-prodrug ratio that provided the desired result at the high dose of prodrug. The PK parameter value of interest (e.g., drug Cmax) is then assessed. If a desired PK parameter value results following ingestion of the single dose unit equivalent, then single dose units that provide for a desired concentration-dose PK profile are identified. For example, where a zero dose linear profile is desired, the drug Cmax following ingestion of a single dose unit does not increase significantly following ingestion of a multiple number of the single dose units.

### Methods for manufacturing, formulating, and packaging dose units

Dose units of the present disclosure can be made using manufacturing methods available in the art and can be of a variety of forms suitable for enteral (including oral, buccal and sublingual) administration, for example as a tablet, capsule, thin film, powder, suspension, solution, syrup, dispersion or emulsion. The dose unit can contain components conventional in pharmaceutical preparations, e.g. one or more carriers, binders, lubricants, excipients (e.g., to impart controlled release characteristics), pH modifiers, flavoring agents (e.g., sweeteners), bulking agents, coloring agents or further active agents. Dose units of the present disclosure can include can include an enteric coating or other component(s) to facilitate protection from stomach acid, where desired.

Dose units can be of any suitable size or shape. The dose unit can be of any shape suitable for enteral administration, e.g., ellipsoid, lenticular, circular, rectangular, cylindrical, and the like.

Dose units provided as dry dose units can have a total weight of from about 1 microgram to about 1 gram, and can be from about 5 micrograms to 1.5 grams, from about 50 micrograms to 1 gram, from about 100 micrograms to 1 gram, from 50 micrograms to 750 milligrams, and may be from about 1 microgram to 2 grams.

Dose units can comprise components in any relative amounts. For example, dose units can be from about 0.1% to 99% by weight of active ingredients (i.e., prodrug and inhibitor) per total weight of dose unit (0.1% to 99% total combined weight of prodrug and inhibitor per total weight of single dose unit). In some embodiments, dose units can be from 10% to 50%, from 20% to 40%, or about 30% by weight of active ingredients per total weight dose unit.

Dose units can be provided in a variety of different forms and optionally provided in a manner suitable for storage. For example, dose units can be disposed within a container suitable for containing a pharmaceutical composition. The container can be, for example, a bottle (e.g., with a closure device, such as a cap), a blister pack (e.g., which can provide for enclosure of one or more dose units per blister), a vial, flexible packaging (e.g., sealed Mylar or plastic bags), an ampule (for single dose units in solution), a dropper, thin film, a tube and the like.

Containers can include a cap (e.g., screw cap) that is removably connected to the container over an opening through which the dose units disposed within the container can be accessed.

Containers can include a seal which can serve as a tamper-evident and/or tamper-resistant element, which seal is disrupted upon access to a dose unit disposed within the container. Such seal elements can be, for example, a frangible element that is broken or otherwise modified upon access to a dose unit disposed within the container. Examples of such frangible seal elements include a seal positioned over a container opening such that access to a dose unit within the container requires disruption of the seal (e.g., by peeling and/or piercing the seal). Examples of frangible seal elements include a frangible ring disposed around a container opening and in connection with a cap such that the ring is broken upon opening of the cap to access the dose units in the container.

Dry and liquid dose units can be placed in a container (e.g., bottle or package, e.g., a flexible bag) of a size and configuration adapted to maintain stability of dose units over a period during which the dose units are dispensed into a prescription. For example, containers can be sized and configured to contain 10, 20, 30, 40, 50, 60, 70, 80, 90, 100 or more single dry or liquid dose units. The containers can be sealed or resealable. The containers can packaged in a carton (e.g., for shipment from a manufacturer to a pharmacy or other dispensary). Such cartons can be boxes, tubes, or of other configuration, and may be made of any material (e.g., cardboard, plastic, and the like). The packaging system and/or containers disposed therein can have one or more affixed labels (e.g., to provide information such as lot number, dose unit type, manufacturer, and the like).

The container can include a moisture barrier and/or light barrier, e.g., to facilitate maintenance of stability of the active ingredients in the dose units contained therein. Where the dose unit is a dry dose unit, the container can include a desiccant pack which is disposed within the container. The container can be adapted to contain a single dose unit or multiples of a dose unit. The container can include a dispensing control mechanism, such as a lock out mechanism that facilitates maintenance of dosing regimen.

The dose units can be provided in solid or semi-solid form, and can be a dry dose unit. "Dry dose unit" refers to a dose unit that is in other than in a completely liquid form. Examples of dry dose units include, for example, tablets, capsules (e.g., solid capsules, capsules containing liquid), thin film, microparticles, granules, powder and the like. Dose units can be provided as liquid dose units, where the dose units can be provided as single or multiple doses of a formulation containing prodrug and inhibitor in liquid form. Single doses of a dry or liquid dose unit can be disposed within a sealed container, and sealed containers optionally provided in a packaging system, e.g., to provide for a prescribed number of doses, to provide for shipment of dose units, and the like.

Dose units can be formulated such that the prodrug and inhibitor are present in the same carrier, e.g., solubilized or suspended within the same matrix. Alternatively, dose units can be composed of two or more portions, where the prodrug and inhibitor can be provided in the same or different portions, and can be provided in adjacent or non-adjacent portions.

Dose units can be provided in a container in which they are disposed, and may be provided as part of a packaging system (optionally with instructions for use). For example, dose units containing different amounts of prodrug can be provided in separate containers, which containers can be disposed with in a larger container (e.g., to facilitate protection of dose units for shipment). For example, one or more dose units as described herein can be provided in separate containers, where dose units of different composition are provided in separate containers, and the separate containers disposed within package for dispensing.

In another example, dose units can be provided in a double-chambered dispenser where a first chamber contains a prodrug formulation and a second chamber contains an inhibitor formulation. The dispenser can be adapted to provide for mixing of a prodrug formulation and an inhibitor formulation prior to ingestion. For example, the two chambers of the dispenser can be separated by a removable wall (e.g., frangible wall) that is broken or removed prior to administration to allow mixing of the formulations of the two chambers. The first and second chambers can terminate into a dispensing outlet, optionally through a common chamber. The formulations can be provided in dry or liquid form, or a combination thereof. For example, the formulation in the first chamber can be liquid and the formulation in the second chamber can be dry, both can be dry, or both can be liquid.

Dose units that provide for controlled release of prodrug, of inhibitor, or of both prodrug and inhibitor are contemplated by the present disclosure, where "controlled release" refers to release of one or both of prodrug and inhibitor from the dose unit over a selected period of time and/or in a pre-selected manner. In embodiments of the present disclosure, dose units provide for controlled immediate release of the prodrug.

### Methods of use of dose units

Dose units are advantageous because they find use in methods to reduce side effects and/or improve tolerability of drugs to patients in need thereof by, for example, limiting a PK parameter as disclosed herein. The present disclosure thus provides methods to reduce side effects by administering a dose unit of the present disclosure to a patient in need so as to provide for a reduction of side effects as compared to those associated with administration of drug and/or as compared to administration of prodrug without inhibitor. The present disclosure also provides methods to improve tolerability of drugs by administering a dose unit of the present disclosure to a patient in need so as to provide for improvement in tolerability as compared to administration of drug and/or as compared to administration of prodrug without inhibitor.

Dose units find use in methods for increasing patient compliance of a patient with a therapy prescribed by a clinician, where such methods involve directing administration of a dose unit described herein to a patient in need of therapy so as to provide for increased patient compliance as compared to a therapy involving administration of drug and/or as compared to administrations of prodrug without inhibitor. Such methods can help increase the likelihood that a clinician-specified therapy occurs as prescribed.

Dose units can provide for enhanced patient compliance and clinician control. For example, by limiting a PK parameter (e.g., such as drug Cmax or drug exposure) when multiples (e.g., two or more, three or more, or four or more) dose units are ingested, a patient requiring a higher dose of drug must seek the assistance of a clinician. The dose units can provide for control of the degree to which a patient can readily "self-medicate", and further can provide for the patient to adjust dose to a dose within a permissible range. Dose units can provide for reduced side effects, by for example, providing for delivery of drug at an efficacious dose but with a modified PK profile over a period of treatment, e.g., as defined by a decreased PK parameter (e.g., decreased drug Cmax, decreased drug exposure).

Dose units find use in methods to reduce the risk of unintended overdose of drug that can follow ingestion of multiple doses taken at the same time or over a short period of time. Such methods of the present disclosure can provide for reduction of risk of unintended overdose as compared to risk of unintended overdose of drug and/or as compared to risk of unintended overdose of prodrug without inhibitor. Such methods involve directing administration of a dosage described herein to a patient in need of drug released by conversion of the prodrug. Such methods can help avoid unintended overdosing due to intentional or unintentional misuse of the dose unit.

The present disclosure provides methods to reduce misuse and abuse of a drug, as well as to reduce risk of overdose, that can accompany ingestion of multiples of doses of a drug, e.g., ingested at the same time. Such methods generally involve combining in a dose unit a prodrug and a trypsin inhibitor that mediates release of drug from the prodrug, where the inhibitor is present in the dose unit in an amount effective to attenuate release of drug from the prodrug, e.g., following ingestion of multiples of dose units by a patient. Such methods provide for a modified concentration-dose PK profile while providing therapeutically effective levels from a single dose unit, as directed by the prescribing clinician. Such methods can provide for, for example, reduction of risks that can accompany misuse and/or abuse of a prodrug, particularly where conversion of the prodrug provides for release of a narcotic or other drug of abuse (e.g., opioid). For example, when the prodrug provides for release of a drug of abuse, dose units can provide for reduction of reward that can follow ingestion of multiples of dose units of a drug of abuse.

Dose units can provide clinicians with enhanced flexibility in prescribing drug. For example, a clinician can prescribe a dosage regimen involving different dose strengths, which can involve two or more different dose units of prodrug and inhibitor having different relative amounts of prodrug, different amounts of inhibitor, or different amounts of both prodrug and inhibitor. Such different strength dose units can provide for delivery of drug according to different PK parameters (e.g., drug exposure, drug Cmax, and the like as described herein). For example, a first dose unit can provide for delivery of a first dose of drug following ingestion, and a second dose unit can provide for delivery of a second dose of drug following ingestion. The first and second prodrug doses of the dose units can be different strengths, e.g., the second dose can be greater than the first dose. A clinician can thus prescribe a collection of two or more, or three or more dose units of different strengths, which can be accompanied by instructions to facilitate a degree of self-medication, e.g., to increase delivery of an opioid drug according to a patient's needs to treat pain.

### Thwarting tampering by trypsin mediated release of amphetamine from prodrug

The disclosure provides for a composition comprising one or more of Compound AM-9 and Compound AM-10 and a trypsin inhibitor that reduces drug abuse potential. A trypsin inhibitor can thwart the ability of a user to apply trypsin to effect the release of amphetamine from the amphetamine prodrug, Compound AM-9 or Compound AM-10, *in vitro.* For example, if an abuser attempts to incubate trypsin with a composition of the embodiments that includes one or more of Compound AM-9 and Compound AM-10 and a trypsin inhibitor, the trypsin inhibitor can reduce the action of the added trypsin, thereby thwarting attempts to release amphetamine for purposes of abuse.

Features of the subject matter described herein may be beneficial alone or in combination, with one or more other features. Again, any references herein to methods of treatment by therapy or surgery or *in vivo* diagnosis are to be interpreted as references to compounds, pharmaceutical compositions and medicaments described herein for use in those methods. Certain non-limiting features are provided below. As will be apparent to those of skill in the art upon reading this disclosure, each of the features may be used or combined with any of the preceding or following features. This is intended to provide support for all such combinations of features and is not limited to combinations of features explicitly provided below:

Described herein is amphetamine-arginine-glycine-acetate, Compound AM-9, shown below: 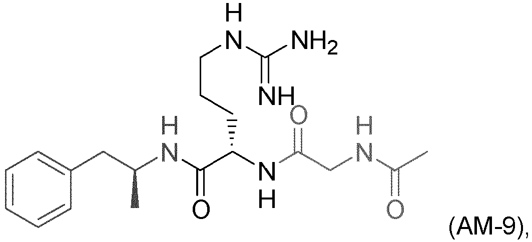 or acceptable salts, solvates, and hydrates thereof.

Also described herein is a composition comprising amphetamine-arginine-glycine-acetate, Compound AM-9, shown below: 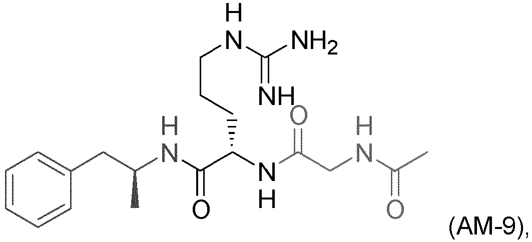 or acceptable salts, solvates, and hydrates thereof.

Also described herein is a method of treating or preventing pain in a patient in need thereof, which comprises administering an effective amount of the compound described hereinabove to the patient.

Also described herein is a compound described hereinabove for use in medical therapy.

Also described herein is a compound described hereinabove for use in the treatment or prevention of attention deficit hyperactivity disorder (ADHD).

Also described herein is a composition comprising a trypsin inhibitor and amphetamine-arginine-glycine-acetate, Compound AM-9, shown below: 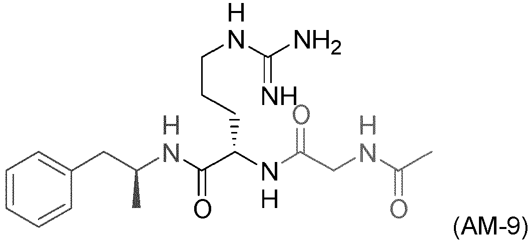 or acceptable salts, solvates, and hydrates thereof.

In one embodiment, the trypsin inhibitor is an arginine mimic or a lysine mimic.

In one embodiment, the arginine mimic or lysine mimic is a synthetic compound.

In one embodiment, the trypsin inhibitor is a compound of formula: 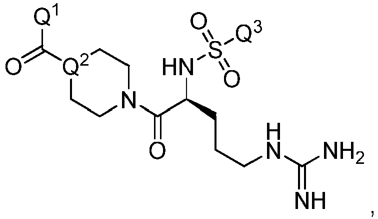 wherein:
Q¹ is selected from -O-Q⁴ or -Q⁴-COOH, where Q⁴ is C₁-C₄ alkyl;
Q² is N or CH; and
Q³ is aryl or substituted aryl.

In one embodiment, the trypsin inhibitor is a compound of formula: 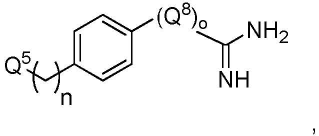 wherein:
Q⁵ is -C(O)-COOH or -NH-Q⁶-Q⁷-SO₂-C₆H₅, where
Q⁶ is -(CH₂)ₚ-COOH;
Q⁷ is -(CH₂)ᵣ-C₆H₅;
Q⁸ is NH;
n is a number from zero to two;
o is zero or one;
p is an integer from one to three; and
r is an integer from one to three.

In one embodiment, the trypsin inhibitor is a compound of formula: 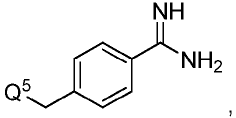 wherein:
Q⁵ is -C(O)-COOH or -NH-Q⁶-Q⁷-SO₂-C₆H₅, where
Q⁶ is -(CH₂)ₚ-COOH;
Q⁷ is -(CH₂)ᵣ-C₆H₅; and
p is an integer from one to three; and
r is an integer from one to three.

In one embodiment, the trypsin inhibitor is a compound of formula: 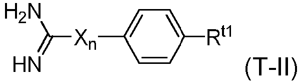 wherein
X is NH;
n is zero or one; and
R^{t1} is selected from hydrogen, halogen, nitro, alkyl, substituted alkyl, alkoxy, carboxyl, alkoxycarbonyl, acyl, aminoacyl, guanidine, amidino, carbamide, amino, substituted amino, hydroxyl, cyano and -(CH₂)ₘ-C(O)-O-(CH₂)ₘ-C(O)-N-Rⁿ¹Rⁿ², wherein each m is independently zero to 2; and Rⁿ¹ and Rⁿ² are independently selected from hydrogen and C₁₋₄ alkyl.

In one embodiment, the trypsin inhibitor is a compound of formula: 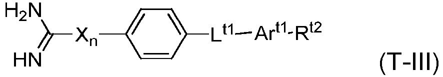 wherein
X is NH;
n is zero or one;
L^{t1} is selected from -C(O)-O- ; -O-C(O)-; -O-(CH₂)ₘ-O-;-OCH₂-Ar^{t2}-CH₂O-; - C(O)-NR^{t3}-; and - NR^{t3}-C(O)-;
R^{t3} is selected from hydrogen, C₁₋₆ alkyl, and substituted C₁₋₆ alkyl;
Ar^{t1} and Ar^{t2} are independently a substituted or unsubstituted aryl group;
m is a number from 1 to 3; and
R^{t2} is selected from hydrogen, halogen, nitro, alkyl, substituted alkyl, alkoxy, carboxyl, alkoxycarbonyl, acyl, aminoacyl, guanidine, amidino, carbamide, amino, substituted amino, hydroxyl, cyano and -(CH₂)ₘ-C(O)-O-(CH₂)ₘ-C(O)-N-Rⁿ¹Rⁿ², wherein each m is independently zero to 2; and Rⁿ¹ and Rⁿ² are independently selected from hydrogen and C₁₋₄ alkyl.

In one embodiment, the trypsin inhibitor is a compound of formula: 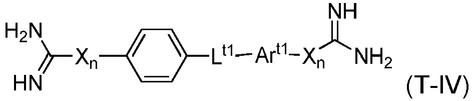 wherein
each X is NH;
each n is independently zero or one;
L^{t1} is selected from -C(O)-O- ; -O-C(O)-; -O-(CH₂)ₘ-O-;-OCH₂-Ar^{t2}-CH₂O-; - C(O)-NR^{t3}-; and - NR^{t3}-C(O)-;
R^{t3} is selected from hydrogen, C₁₋₆ alkyl, and substituted C₁₋₆ alkyl;
Ar^{t1} and Ar^{t2} are independently a substituted or unsubstituted aryl group; and
m is a number from 1 to 3.

In one embodiment, the trypsin inhibitor is selected from
(S)-ethyl 4-(5-guanidino-2-(naphthalene-2-sulfonamido)pentanoyl)piperazine-1-carboxylate;
(S)-ethyl 4-(5-guanidino-2-(2,4,6-triisopropylphenylsulfonamido)pentanoyl)piperazine-1-carboxylate;
(S)-ethyl 1-(5-guanidino-2-(naphthalene-2-sulfonamido)pentanoyl)piperidine-4-carboxylate;
(S)-ethyl 1-(5-guanidino-2-(2,4,6-triisopropylphenylsulfonamido)pentanoyl)piperidine-4-carboxylate;
(S)-6-(4-(5-guanidino-2-(naphthalene-2-sulfonamido)pentanoyl)piperazin-1-yl)-6-oxohexanoic acid;
4-aminobenzimidamide;
3-(4-carbamimidoylphenyl)-2-oxopropanoic acid;
(S)-5-(4-carbamimidoylbenzylamino)-5-oxo-4-((R)-4-phenyl-2-(phenylmethylsulfonamido)butanamido)pentanoic acid;
6-carbamimidoylnaphthalen-2-yl 4-(diaminomethyleneamino)benzoate; and
4,4'-(pentane-1,5-diylbis(oxy))dibenzimidamide.

**In** one embodiment, the trypsin inhibitor is Compound 109.

Described herein is a method of treating or preventing attention deficit hyperactivity disorder (ADHD) in a patient in need thereof, which comprises administering an effective amount of a composition described hereinabove to the patient.

Described herein is a composition described hereinabove for use in medical therapy.

Described herein is a composition described hereinabove for use in the treatment or prevention of attention deficit hyperactivity disorder (ADHD).

Described herein is a method for reducing drug abuse potential of a composition containing a compound of described hereinabove, the method comprising: combining Compound AM-9 with a trypsin inhibitor, wherein the trypsin inhibitor reduces the ability of a user to release amphetamine from Compound AM-9 by addition of trypsin.

Described herein is a composition comprising:
a prodrug comprising amphetamine covalently bound to a promoiety comprising a trypsin-cleavable moiety, wherein cleavage of the trypsin-cleavable moiety by trypsin mediates release of amphetamine, wherein the prodrug is Compound AM-9; and
a trypsin inhibitor that interacts with the trypsin that mediates enzymatically-controlled release of amphetamine from the prodrug following ingestion of the composition.

Descirbed herein is a dose unit comprising the composition described , wherein
the prodrug and trypsin inhibitor are present in the dose unit in an amount effective to provide for a pre-selected pharmacokinetic (PK) profile following ingestion.

In one embodiment, the pre-selected PK profile comprises at least one PK parameter value that is less than the PK parameter value of amphetamine released following ingestion of an equivalent dosage of the prodrug in the absence of inhibitor.

In one embodiment, the PK parameter value is selected from amphetamine Cmax value, amphetamine exposure value, and a (1/ amphetamine Tmax) value.

In one embodiment, the dose unit provides for a pre-selected PK profile following ingestion of at least two dose units.

In one embodiment, the pre-selected PK profile is modified relative to the PK profile following ingestion of an equivalent dosage of the prodrug in the absence of inhibitor.

In one embodiment, the dose unit provides that ingestion of an increasing number of the dose units provides for a linear PK profile.

In one embodiment, the dose unit provides that ingestion of an increasing number of the dose units provides for a nonlinear PK profile.

In one embodiment, the PK parameter value is selected from a amphetamine Cmax value, a (1/amphetamine Tmax) value, and amphetamine exposure value.

Described herein is a method to treat a patient comprising administering a composition or dose unit described hereinabove to a patient in need thereof.

Described herein is a method of making a dose unit, the method comprising: combining in a dose unit:
a prodrug comprising amphetamine covalently bound to a promoiety comprising a trypsin-cleavable moiety, wherein cleavage of the trypsin-cleavable moiety by trypsin mediates release of amphetamine, wherein the prodrug is Compound AM-9; and
a trypsin inhibitor that interacts with the trypsin that mediates enzymatically-controlled release of amphetamine from the prodrug;
wherein the prodrug and trypsin inhibitor are present in the dose unit in an amount effective to attenuate release of amphetamine from the prodrug such that ingestion of multiples of dose units by a patient does not provide a proportional release of amphetamine.

In one embodiment, said release of amphetamine is decreased compared to release of amphetamine by an equivalent dosage of prodrug in the absence of inhibitor.

Described herein is a method for identifying a prodrug and a trypsin inhibitor suitable for formulation in a dose unit, the method comprising:
combining a prodrug, a trypsin inhibitor, and trypsin in a reaction mixture, wherein the prodrug comprises amphetamine covalently bound to a promoiety comprising a trypsin-cleavable moiety, wherein cleavage of the trypsin-cleavable moiety by trypsin mediates release of amphetamine, wherein the prodrug is Compound AM-9; and
detecting prodrug conversion,
wherein a decrease in prodrug conversion in the presence of the trypsin inhibitor as compared to prodrug conversion in the absence of the trypsin inhibitor indicates the prodrug and trypsin inhibitor are suitable for formulation in a dose unit.

Described herein is a method for identifying a prodrug and a trypsin inhibitor suitable for formulation in a dose unit, the method comprising:
administering to an animal a prodrug and a trypsin inhibitor, wherein the prodrug comprises amphetamine covalently bound to a promoiety comprising a trypsin-cleavable moiety, wherein cleavage of the trypsin-cleavable moiety by trypsin mediates release of amphetamine, wherein the prodrug is Compound AM-9; and
detecting prodrug conversion, wherein a decrease in amphetamine conversion in the presence of the trypsin inhibitor as compared to amphetamine conversion in the absence of the trypsin inhibitor indicates the prodrug and trypsin inhibitor are suitable for formulation in a dose unit.

In one embodiment, said administering comprises administering to the animal increasing doses of inhibitor co-dosed with a selected fixed dose of prodrug.

In one embodiment, said detecting facilitates identification of a dose of inhibitor and a dose of prodrug that provides for a pre-selected pharmacokinetic (PK) profile.

In one embodiment, said method comprises an *ex vivo* assay.

Described herein is a method for identifying a prodrug and a trypsin inhibitor suitable for formulation in a dose unit, the method comprising:
administering to an animal tissue a prodrug and a trypsin inhibitor, wherein the prodrug comprises amphetamine covalently bound to a promoiety comprising a trypsin-cleavable moiety, wherein cleavage of the trypsin-cleavable moiety by trypsin mediates release of amphetamine, wherein the prodrug is Compound AM-9; and
detecting prodrug conversion, wherein a decrease in prodrug conversion in the presence of the trypsin inhibitor as compared to prodrug conversion in the absence of the trypsin inhibitor indicates the prodrug and trypsin inhibitor are suitable for formulation in a dose unit.

Described herein is a method comprising administering to a subject a therapeutically effective amount of amphetamine-arginine-glycine-acetate, Compound AM-9, shown below: 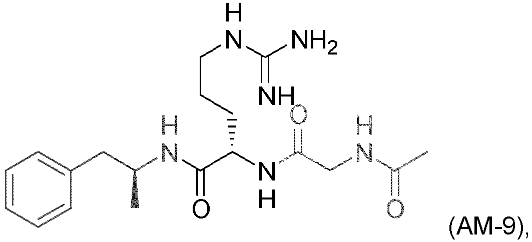 or acceptable salts, solvates, and hydrates thereof,
in a manner sufficient to provide a peak plasma concentration in the subject 60 minutes or less after Compound AM-9 is administered to the subject.

In one embodiment, the method comprises administering Compound AM-9 in a manner sufficient to provide a peak plasma concentration in the subject 30 minutes or less after Compound AM-9 is administered to the subject.

In one embodiment, the method comprises administering Compound AM-9 in a manner sufficient to provide a peak plasma concentration in the subject 15 minutes or less after Compound AM-9 is administered to the subject.

Described herein is an amphetamine-arginine-alanine-acetate, Compound AM-10, shown below: 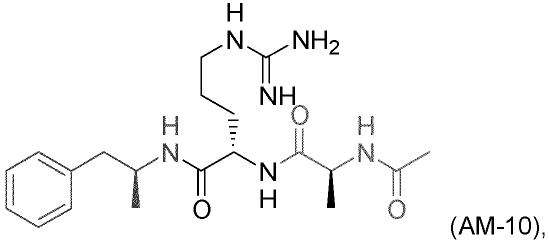 or acceptable salts, solvates, and hydrates thereof.

Descirbed herein is a composition comprising amphetamine-arginine-alanine-acetate, Compound AM-10, shown below: 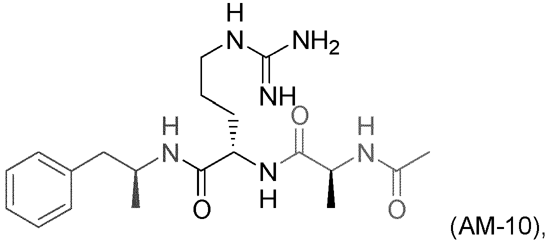 or acceptable salts, solvates, and hydrates thereof.

Described herein is a method of treating or preventing pain in a patient in need thereof, which comprises administering an effective amount of a compound described hereinabove to the patient.

Described herein is a compound described hereinabove for use in medical therapy.

Descirbed herein is a compound described hereinabove for use in the treatment or prevention of attention deficit hyperactivity disorder (ADHD).

Described herein is a composition comprising a trypsin inhibitor and amphetamine-arginine-alanine-acetate, Compound AM-10, shown below: 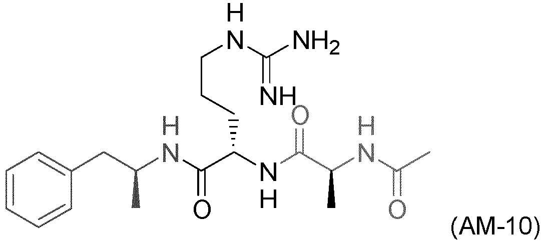 or acceptable salts, solvates, and hydrates thereof.

In one embodiment, the trypsin inhibitor is an arginine mimic or a lysine mimic.

In one embodiment, the arginine mimic or lysine mimic is a synthetic compound.

In one embodiment, the trypsin inhibitor is a compound of formula: 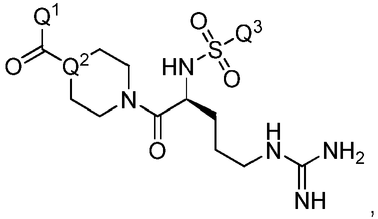 wherein:
Q¹ is selected from -O-Q⁴ or -Q⁴-COOH, where Q⁴ is C₁-C₄ alkyl;
Q² is N or CH; and
Q³ is aryl or substituted aryl.

In one embodiment, the trypsin inhibitor is a compound of formula: 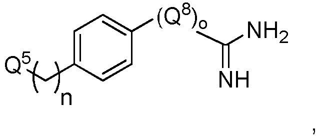 wherein:
Q⁵ is -C(O)-COOH or -NH-Q⁶-Q⁷-SO₂-C₆H₅, where
Q⁶ is -(CH₂)ₚ-COOH;
Q⁷ is -(CH₂)ᵣ-C₆H₅;
Q⁸ is NH;
n is a number from zero to two;
o is zero or one;
p is an integer from one to three; and
r is an integer from one to three.

In one embodiment, the trypsin inhibitor is a compound of formula: 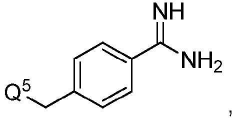 wherein:
Q⁵ is -C(O)-COOH or -NH-Q⁶-Q⁷-SO₂-C₆H₅, where
Q⁶ is -(CH₂)ₚ-COOH;
Q⁷ is -(CH₂)ᵣ-C₆H₅; and
p is an integer from one to three; and
r is an integer from one to three.

In one embodiment, the trypsin inhibitor is a compound of formula: 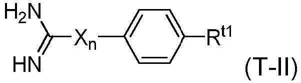 wherein
X is NH;
n is zero or one; and
R^{t1} is selected from hydrogen, halogen, nitro, alkyl, substituted alkyl, alkoxy, carboxyl, alkoxycarbonyl, acyl, aminoacyl, guanidine, amidino, carbamide, amino, substituted amino, hydroxyl, cyano and -(CH₂)ₘ-C(O)-O-(CH₂)ₘ-C(O)-N-Rⁿ¹Rⁿ², wherein each m is independently zero to 2; and Rⁿ¹ and Rⁿ² are independently selected from hydrogen and C₁₋₄ alkyl.

In one embodiment, the trypsin inhibitor is a compound of formula: 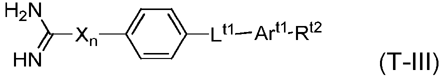 wherein
X is NH;
n is zero or one;
L^{t1} is selected from -C(O)-O- ; -O-C(O)-; -O-(CH₂)ₘ-O-;-OCH₂-Ar^{t2}-CH₂O-; - C(O)-NR^{t3}-; and - NR^{t3}-C(O)-;
R^{t3} is selected from hydrogen, C₁₋₆ alkyl, and substituted C₁₋₆ alkyl;
Ar^{t1} and Ar^{t2} are independently a substituted or unsubstituted aryl group;
m is a number from 1 to 3; and
R^{t2} is selected from hydrogen, halogen, nitro, alkyl, substituted alkyl, alkoxy, carboxyl, alkoxycarbonyl, acyl, aminoacyl, guanidine, amidino, carbamide, amino, substituted amino, hydroxyl, cyano and -(CH₂)ₘ-C(O)-O-(CH₂)ₘ-C(O)-N-Rⁿ¹Rⁿ², wherein each m is independently zero to 2; and Rⁿ¹ and Rⁿ² are independently selected from hydrogen and C₁₋₄ alkyl.

In one embodiment, the trypsin inhibitor is a compound of formula: 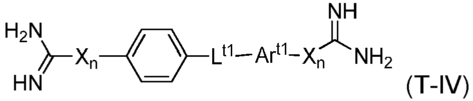 wherein
each X is NH;
each n is independently zero or one;
L^{t1} is selected from -C(O)-O- ; -O-C(O)-; -O-(CH₂)ₘ-O-;-OCH₂-Ar^{t2}-CH₂O-; - C(O)-NR^{t3}-; and - NR^{t3}-C(O)-;
R^{t3} is selected from hydrogen, C₁₋₆ alkyl, and substituted C₁₋₆ alkyl;
Ar^{t1} and Ar^{t2} are independently a substituted or unsubstituted aryl group; and
m is a number from 1 to 3.

In one embodiment, the trypsin inhibitor is selected from
(S)-ethyl 4-(5-guanidino-2-(naphthalene-2-sulfonamido)pentanoyl)piperazine-1-carboxylate;
(S)-ethyl 4-(5-guanidino-2-(2,4,6-triisopropylphenylsulfonamido)pentanoyl)piperazine-1 -carboxylate;
(S)-ethyl 1-(5-guanidino-2-(naphthalene-2-sulfonamido)pentanoyl)piperidine-4-carboxylate;
(S)-ethyl 1-(5-guanidino-2-(2,4,6-triisopropylphenylsulfonamido)pentanoyl)piperidine-4-carboxylate;
(S)-6-(4-(5-guanidino-2-(naphthalene-2-sulfonamido)pentanoyl)piperazin-1-yl)-6-oxohexanoic acid;
4-aminobenzimidamide;
3-(4-carbamimidoylphenyl)-2-oxopropanoic acid;
(S)-5-(4-carbamimidoylbenzylamino)-5-oxo-4-((R)-4-phenyl-2-(phenylmethylsulfonamido)butanamido)pentanoic acid;
6-carbamimidoylnaphthalen-2-yl 4-(diaminomethyleneamino)benzoate; and
4,4'-(pentane-1,5-diylbis(oxy))dibenzimidamide.

In one embodiment, the trypsin inhibitor is Compound 109.

Described herein is a method of treating or preventing attention deficit hyperactivity disorder (ADHD) in a patient in need thereof, which comprises administering an effective amount of a composition described hereinabove to the patient.

Described herein is a composition described hereinabove for use in medical therapy.

Described herein is a composition described hereinabove for use in the treatment or prevention of attention deficit hyperactivity disorder (ADHD).

Described herein is a method for reducing drug abuse potential of a composition containing a compound described hereinabove, the method comprising: combining Compound AM-9 with a trypsin inhibitor, wherein the trypsin inhibitor reduces the ability of a user to release amphetamine from Compound AM-9 by addition of trypsin.

Described herein is a composition comprising:
a prodrug comprising amphetamine covalently bound to a promoiety comprising a trypsin-cleavable moiety, wherein cleavage of the trypsin-cleavable moiety by trypsin mediates release of amphetamine, wherein the prodrug is Compound AM-10; and
a trypsin inhibitor that interacts with the trypsin that mediates enzymatically-controlled release of amphetamine from the prodrug following ingestion of the composition.

Described herein is a dose unit comprising the composition described hereinabove, wherein
the prodrug and trypsin inhibitor are present in the dose unit in an amount effective to provide for a pre-selected pharmacokinetic (PK) profile following ingestion.

In one embodiment, the pre-selected PK profile comprises at least one PK parameter value that is less than the PK parameter value of amphetamine released following ingestion of an equivalent dosage of the prodrug in the absence of inhibitor.

In one embodiment, the PK parameter value is selected from amphetamine Cmax value, amphetamine exposure value, and a (1/amphetamine Tmax) value.

In one embodiment, the dose unit provides for a pre-selected PK profile following ingestion of at least two dose units.

In one embodiment, the pre-selected PK profile is modified relative to the PK profile following ingestion of an equivalent dosage of the prodrug in the absence of inhibitor.

In one embodiment, the dose unit provides that ingestion of an increasing number of the dose units provides for a linear PK profile.

In one embodiment, the dose unit provides that ingestion of an increasing number of the dose units provides for a nonlinear PK profile.

In one embodiment, the PK parameter value is selected from a amphetamine Cmax value, a (1/amphetamine Tmax) value, and amphetamine exposure value.

Described herein is a method to treat a patient comprising administering a composition or dose unit described hereinabove to a patient in need thereof.

Described herein is a method of making a dose unit, the method comprising: combining in a dose unit:
a prodrug comprising amphetamine covalently bound to a promoiety comprising a trypsin-cleavable moiety, wherein cleavage of the trypsin-cleavable moiety by trypsin mediates release of amphetamine, wherein the prodrug is Compound AM-10; and
a trypsin inhibitor that interacts with the trypsin that mediates enzymatically-controlled release of amphetamine from the prodrug;
wherein the prodrug and trypsin inhibitor are present in the dose unit in an amount effective to attenuate release of amphetamine from the prodrug such that ingestion of multiples of dose units by a patient does not provide a proportional release of amphetamine.

In one embodiment, said release of amphetamine is decreased compared to release of amphetamine by an equivalent dosage of prodrug in the absence of inhibitor.

Described herein is a method for identifying a prodrug and a trypsin inhibitor suitable for formulation in a dose unit, the method comprising:
combining a prodrug, a trypsin inhibitor, and trypsin in a reaction mixture, wherein the prodrug comprises amphetamine covalently bound to a promoiety comprising a trypsin-cleavable moiety, wherein cleavage of the trypsin-cleavable moiety by trypsin mediates release of amphetamine, wherein the prodrug is Compound AM-10; and
detecting prodrug conversion,
wherein a decrease in prodrug conversion in the presence of the trypsin inhibitor as compared to prodrug conversion in the absence of the trypsin inhibitor indicates the prodrug and trypsin inhibitor are suitable for formulation in a dose unit.

Described herein is a method for identifying a prodrug and a trypsin inhibitor suitable for formulation in a dose unit, the method comprising:
administering to an animal a prodrug and a trypsin inhibitor, wherein the prodrug comprises amphetamine covalently bound to a promoiety comprising a trypsin-cleavable moiety, wherein cleavage of the trypsin-cleavable moiety by trypsin mediates release of amphetamine, wherein the prodrug is Compound AM-10; and
detecting prodrug conversion, wherein a decrease in amphetamine conversion in the presence of the trypsin inhibitor as compared to amphetamine conversion in the absence of the trypsin inhibitor indicates the prodrug and trypsin inhibitor are suitable for formulation in a dose unit.

In one embodiment, said administering comprises administering to the animal increasing doses of inhibitor co-dosed with a selected fixed dose of prodrug.

In one embodiment, said detecting facilitates identification of a dose of inhibitor and a dose of prodrug that provides for a pre-selected pharmacokinetic (PK) profile.

In one embodiment, said method comprises an *ex vivo* assay.

Described herein is a method for identifying a prodrug and a trypsin inhibitor suitable for formulation in a dose unit, the method comprising:
administering to an animal tissue a prodrug and a trypsin inhibitor, wherein the prodrug comprises amphetamine covalently bound to a promoiety comprising a trypsin-cleavable moiety, wherein cleavage of the trypsin-cleavable moiety by trypsin mediates release of amphetamine, wherein the prodrug is Compound AM-10; and
detecting prodrug conversion, wherein a decrease in prodrug conversion in the presence of the trypsin inhibitor as compared to prodrug conversion in the absence of the trypsin inhibitor indicates the prodrug and trypsin inhibitor are suitable for formulation in a dose unit.

Described herein is a method comprising administering to a subject a therapeutically effective amount of amphetamine-arginine-glycine-acetate, Compound AM-10, shown below: 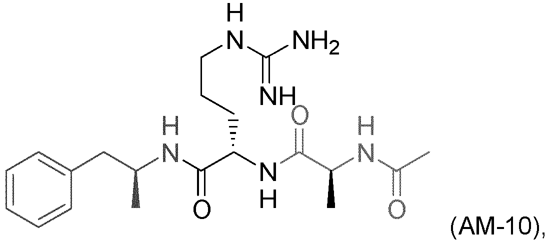 or acceptable salts, solvates, and hydrates thereof,
in a manner sufficient to provide a peak plasma concentration in the subject 60 minutes or less after Compound AM-9 is administered to the subject.

In one embodiment, the method comprises administering Compound AM-10 in a manner sufficient to provide a peak plasma concentration in the subject 30 minutes or less after Compound AM-10 is administered to the subject.

In one embodiment, the method comprises administering Compound AM-9 in a manner sufficient to provide a peak plasma concentration in the subject 15 minutes or less after Compound AM-10 is administered to the subject.

### Examples

The following examples are put forth so as to provide those of ordinary skill in the art with a complete disclosure and description of how to make and use the embodiments, and are not intended to limit the scope of what the inventors regard as their invention nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Celsius, and pressure is at or near atmospheric. Standard abbreviations may be used.

### Biological Data

### Example 1: Pharmacokinetics of Compound AM-9 following oral (PO) administration to rats

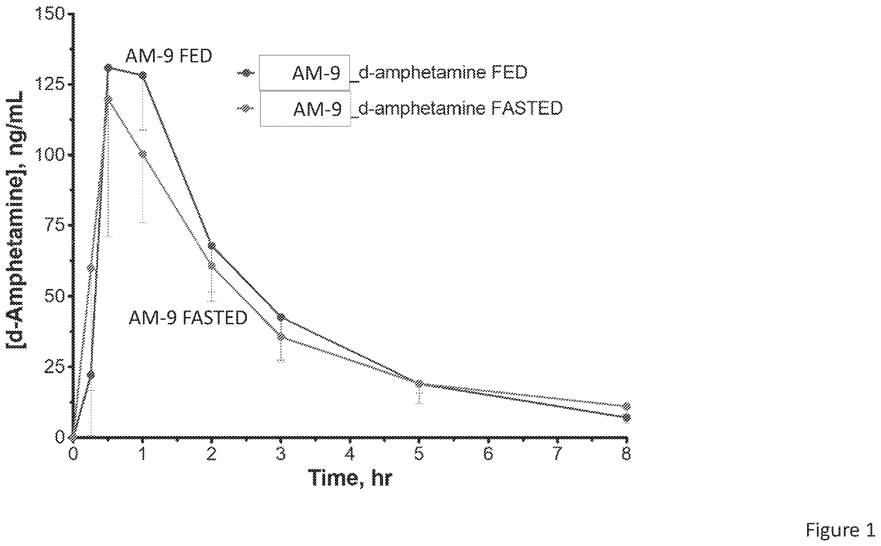

Figure 1 compares mean plasma concentrations over time of amphetamine following PO administration of Compound AM-9 to rats. This example provides a comparison of the immediate release of amphetamine into plasma when Compound AM-9 at a dosage of 10 mg/kg is administered PO to fasted rats and fed male SD rats.

Compound AM-9 was prepared in reverse osmosis (RO) water with 0.1% formic acid. The formulation was a clear solution. Animals were either fasted overnight through 4 hours postdose or fed ad lib prior to dosing, 4 animals per groups. Individual doses of AM-9 was calculated based on body weights taken on the day of dosing. The liquid formulation was administered via oral gavage at 10 mg/kg. Prior to withdrawing the gavage tube, the tube was flushed with approximately 2 mL of water.

Blood was collected from a jugular vein into tubes containing K₂EDTA anticoagulant. Blood (approximately 0.5 mL) was collected from each animal predose and at 0.25, 0.5, 1, 2, 3, 5, and 8 hours postdose and processed for plasma. Plasma samples were evaluated for amphetamine by LC-MS-MS Mass spectrometer (API 5000 AB Sciex Instruments).

### Example 2: Pharmacokinetics of Compound AM-9 following PO administration to rats

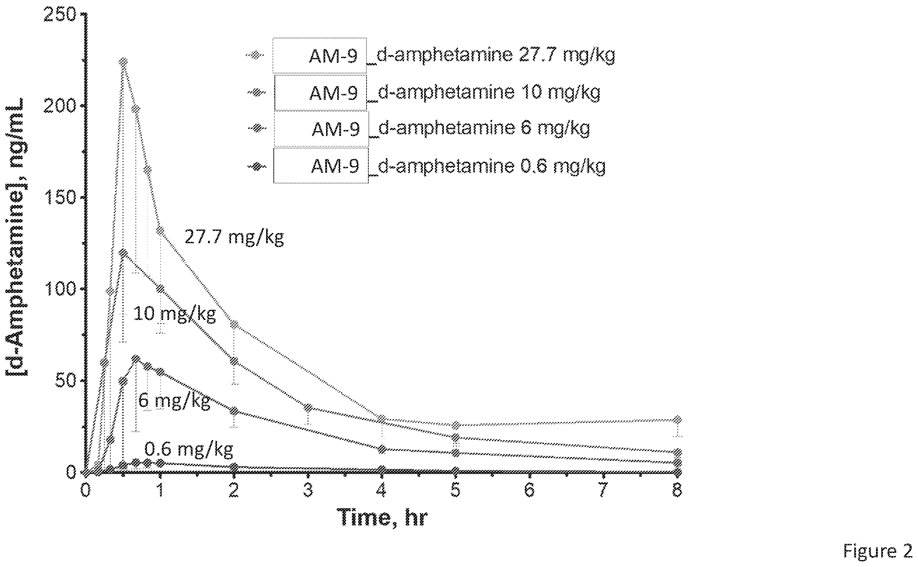

Figure 2 compares mean plasma concentrations over time of amphetamine release following PO administration of different doses of Compound AM-9 to rats. This example depicts the mean plasma concentrations following PO administration to rats at doses of 27.7 mg/kg, 10 mg/kg, 6 mg/kg and 0.6 mg/kg of Compound AM-9. This example provides a comparison of the immediate release of amphetamine into plasma when Compound AM-9 at a dosage of 10 mg/kg is administered PO to fasted rats and fed rats.

Compound AM-9 was prepared in reverse osmosis (RO) water with 0.1% formic acid. The formulation was a clear solution. Animals were fasted overnight through 4 hours postdose prior to dosing, 4 animals per groups. Individual doses of AM-9 was calculated based on body weights taken on the day of dosing. The liquid formulation was administered via oral gavage at 27.7 mg/kg, 10 mg/kg, 6 mg/kg and 0.6 mg/kg of Compound AM-9. Prior to withdrawing the gavage tube, the tube was flushed with approximately 2 mL of water.

Blood was collected from a jugular vein into tubes containing K₂EDTA anticoagulant. Blood (approximately 1 mL) was collected from each animal predose and at 0.167, 0.333, 0.5, 0.667, 0.833 1, 2, 4, 5, and 8 hours postdose and processed for plasma. Plasma samples were evaluated for amphetamine by LC-MS-MS Mass spectrometer (API 5000 AB Sciex Instruments).

### Example 3: Pharmacokinetics of Compound AM-9 and Ampetamine release following PO administration of AM-9 to rats

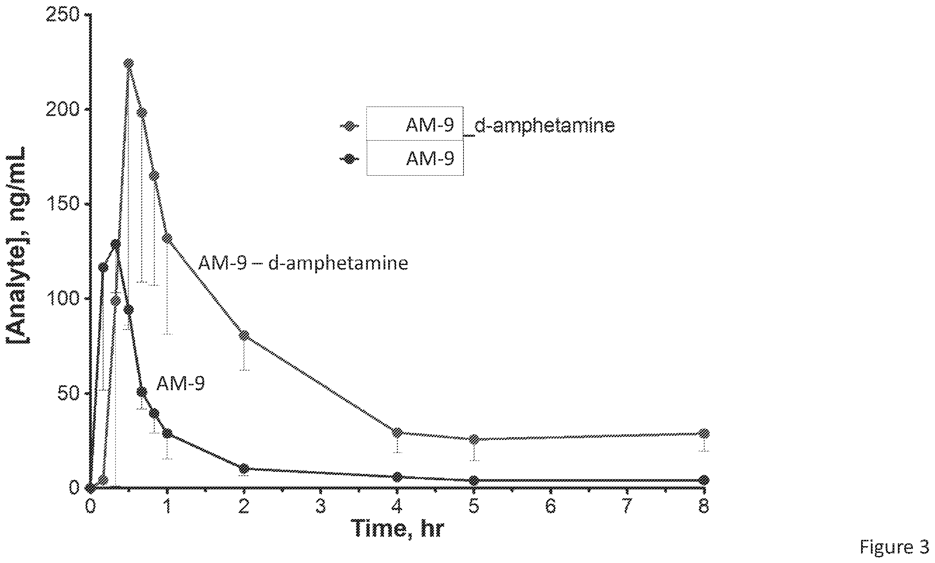

Figure 3 compares mean plasma concentrations over time of AM-9 and analyte amphetamine following PO administration of Compound AM-9 to rats. As shown in Figure 3, the peak plasma concentration (Cmax) of AM-9 at 20 min (Tmax) is approximately half of that of the peak plasma concentration of amphetamine. The amphetamine peak plasma concentration was observed between 20 min and 30 min and demonstrates that the majority of the administered AM-9 is immediately converted to amphetiamine.

Compound AM-9 was prepared in reverse osmosis (RO) water with 0.1% formic acid. The formulation was a clear solution. Animals were fasted overnight through 4 hours postdose prior to dosing, 4 animals per groups. Individual doses of AM-9 was calculated based on body weights taken on the day of dosing. The liquid formulation was administered via oral gavage at 27.7 mg/kg, of Compound AM-9. Prior to withdrawing the gavage tube, the tube was flushed with approximately 2 mL of water.

Blood was collected from a jugular vein into tubes containing K₂EDTA anticoagulant. Blood (approximately 1 mL) was collected from each animal predose and at 0.167, 0.333, 0.5, 0.667, 0.833 1, 2, 4, 5, and 8 hours postdose and processed for plasma. Plasma samples were evaluated for AM-9 and amphetamine by LC-MS-MS Mass spectrometer (API 5000 AB Sciex Instruments).

### Example 4: Pharmacokinetics of Compound AM-9 following PO administration to rats

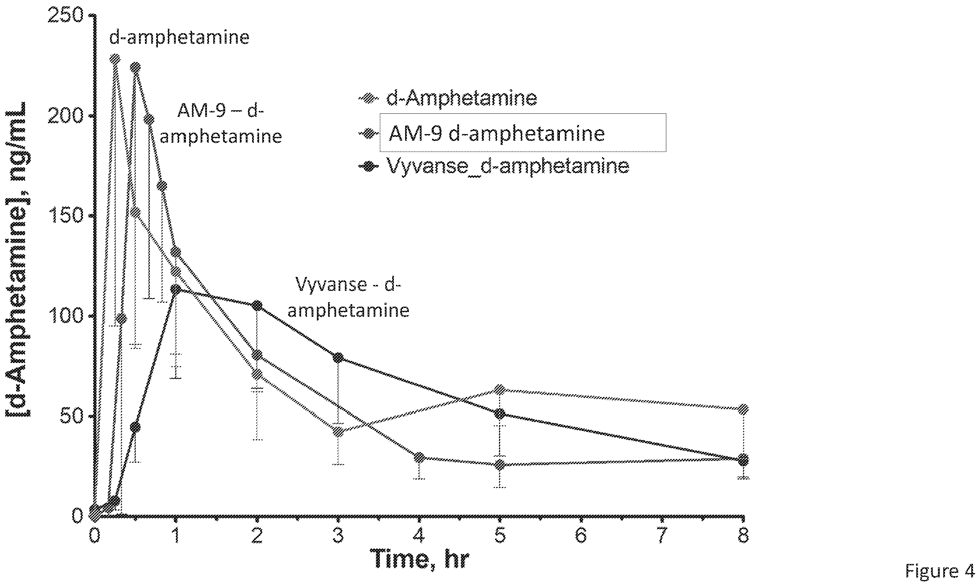

Figure 4 compares mean plasma concentrations over time of amphetamine following PO administration of 1) d-amphetamine 10 mg/kg; 2) Compound AM-9 27.7 mg/kg and 3) Vyvanse to rats. As shown in Figure 4, the peak plasma concentration (Cmax) of amphetamine from release from Compound AM-9 is at 0.5 hr (Tmax) about 15 minutes after peak plasma from direct oral administration of amphetamine (Tmax 0.25 hr). Vyvanase is an extended release prodrug of amphetamine that exhibits much later peak plasma amphetamine concentration (Tmax 1 to 2 hr).

Compound AM-9 was prepared in reverse osmosis (RO) water with 0.1% formic acid. The formulation was a clear solution. Animals were fasted overnight through 4 hours postdose prior to dosing, 4 animals per groups. Individual doses of AM-9 (0.4 mg/kg) was calculated based on body weights taken on the day of dosing. The liquid formulation was administered via oral gavage at 27.7 mg/kg, 10 mg/kg, 6 mg/kg and 0.6 mg/kg of Compound AM-9. Prior to withdrawing the gavage tube, the tube was flushed with approximately 2 mL of water. Amphetamine was administered at 10 mg/kg and Vyvanse was administered at 22 mg/kg.

Blood was collected from a jugular vein into tubes containing K₂EDTA anticoagulant. Blood (approximately 1 mL) was collected from each animal predose and at 0.167, 0.333, 0.5, 0.667, 0.833 1, 2, 4, 5, and 8 hours postdose and processed for plasma. Plasma samples were evaluated for amphetamine by LC-MS-MS Mass spectrometer (API 5000 AB Sciex Instruments).

### Example 5: Pharmacokinetics of Amphetamine and Compound AM-9 following PO administration to dogs

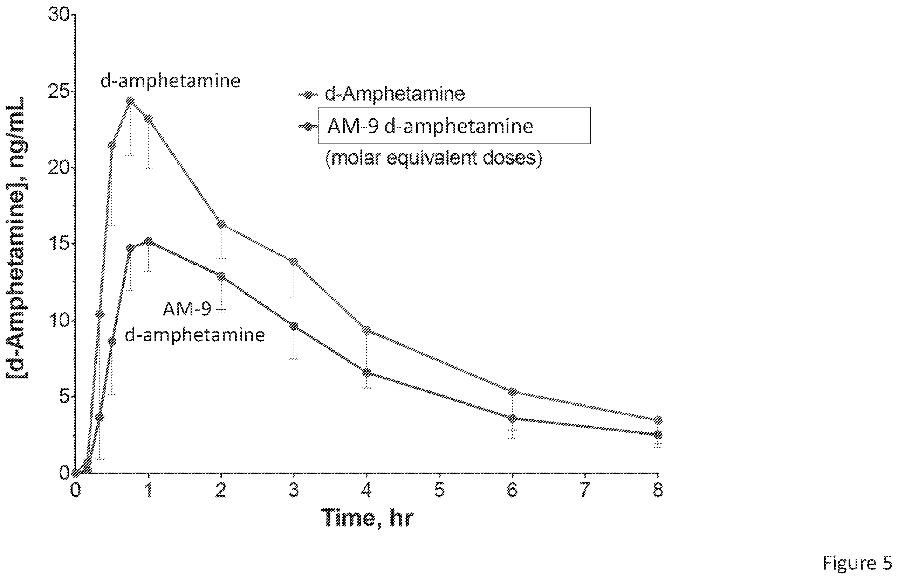

Figure 5 compares mean plasma concentrations over time of amphetamine following PO administration of amphetamine and Compound AM-9 to dogs. This example provides a comparison of amphetamine plasma concentration Cmax and Tmax from administration of amphetamine and amphetamine released from Compound AM-9 which shows the same Tmax (time to maximum plasma concentration) from both amphetamine and from Compound AM-9 when administered PO in dogs.

Male Beagle dogs were fasted overnight through 4 hours postdose. Individual doses of all amphetamine and AM-9 (0.4mg/kg) were calculated based on body weights taken on the day of each dosing. The liquid formulations were administered via oral gavage, 4 per group. Prior to withdrawing the gavage tube, the tube was flushed with approximately 10 mL of water.

Blood (approximately 2 mL) was collected from each animal predose and at 0.167, 0.333, 0.5, 0.75, 1, 2, 3, 4, 6, and 8 hours postdose and processed for plasma. Plasma samples were evaluated for amphetamine by LC-MS-MS Mass spectrometer (API 5000 AB Sciex Instruments).

### Example 6: Pharmacokinetics of Compound AM-9 and Amphetamine following PO administration of AM-9 to dogs

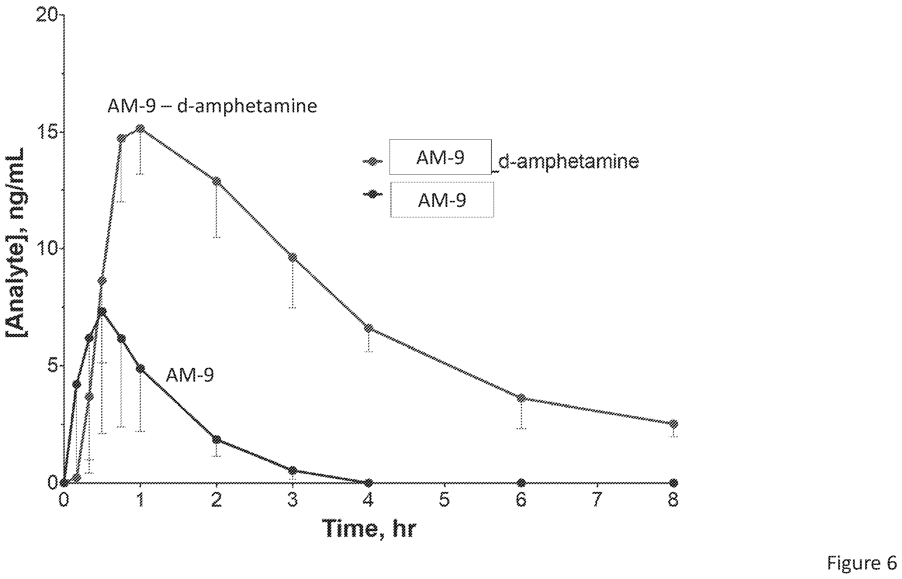

Figure 6 compares mean plasma concentrations over time of AM-9 and analyte amphetamine following PO administration of Compound AM-9 to dogs. As shown in Figure 6, the peak plasma concentration of AM-9 is less than half that of amphetamine. Amphetamine released from AM-9 exhibits a peak plasma concentration within 20 minutes of peak plasma concentration of Compound AM-9 and demonstrates that the majority of the administered AM-9 is immediately converted to amphetiamine.

Male Beagle dogs were fasted overnight through 4 hours postdose. Individual doses of AM-9 were calculated based on body weights taken on the day of each dosing. The liquid formulations were administered via oral gavage, 4 per group. Prior to withdrawing the gavage tube, the tube was flushed with approximately 10 mL of water.

Blood (approximately 2 mL) was collected from each animal predose and at 0.167, 0.333, 0.5, 0.75, 1, 2, 3, 4, 6, and 8 hours postdose and processed for plasma. Plasma samples were evaluated for AM-9 and amphetamine by LC-MS-MS Mass spectrometer (API 5000 AB Sciex Instruments).

### Example 7: Pharmacokinetics of Compound AM-9 following PO administration to dogs

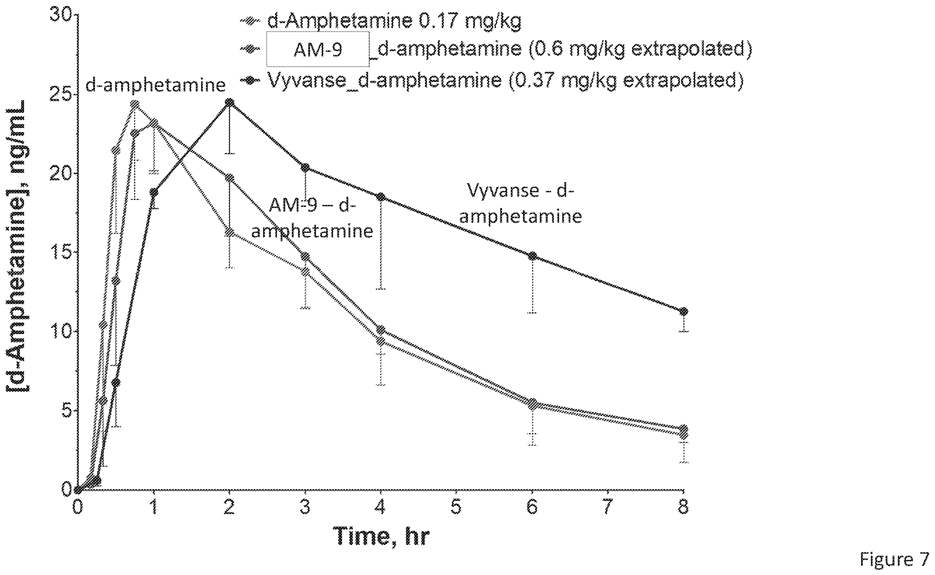

Figure 7 compares mean plasma concentrations over time of amphetamine following PO administration of 1) d-amphetamine; 2) Compound AM-9 and 3) Vyvanse to dogs. As shown in Figure 6, the Tmax for peak plasma concentration of amphetamine from release from Compound AM-9 the same as that from direct administration of amphetamine. Vyvanase is an extended release prodrug of amphetamine that exhibits much later Tmax for peak plasma amphetamine concentration at 2 hr.

Male Beagle dogs were fasted overnight through 4 hours postdose. Individual doses of amphetamine, AM-9 and Vyvanse were calculated based on body weights taken on the day of each dosing. The liquid formulations were administered via oral gavage, 4 per group. Prior to withdrawing the gavage tube, the tube was flushed with approximately 10 mL of water.

Blood (approximately 2 mL) was collected from each animal predose and at 0.167, 0.333, 0.5, 0.75, 1, 2, 3, 4, 6, and 8 hours postdose and processed for plasma. Plasma samples were evaluated for amphetamine by LC-MS-MS Mass spectrometer (API 5000 AB Sciex Instruments).

### Example 8: Pharmacokinetics of Compound AM-9 following PO administration to rats without and with trypsin inhibitor Nafamostat.

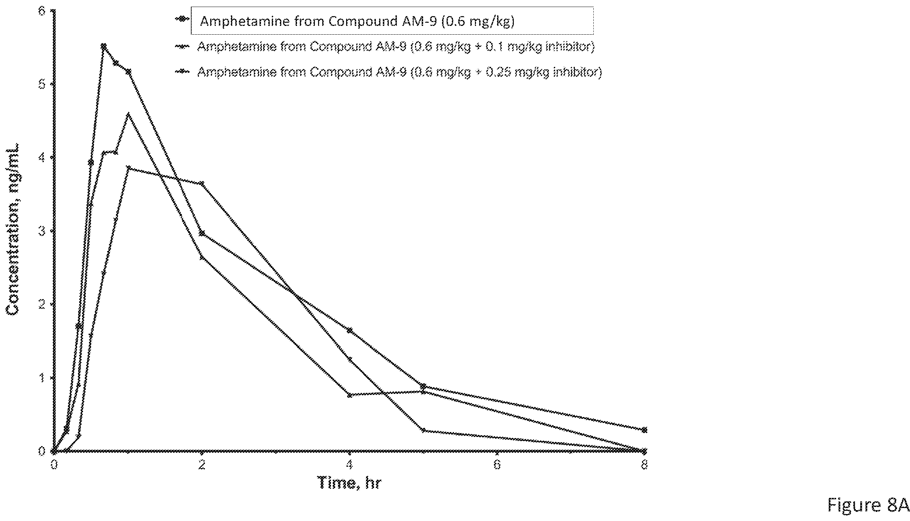

Figures 8A-8B compare mean plasma concentrations over time of amphetamine following PO administration of Compound AM-9 to rats in the absence and presence of trypsin inhibitor nafamostat. This example provides a comparison of the immediate release of amphetamine into plasma when Compound AM-9 at a dosage of 0.6 mg/kg is administered PO to fasted rats alone, or with 0.1 and 0.25 mg/kg trypsin inhibitor Nafamostat. The data shows that Nafamostat reduces the Cmax and increases the Tmax for the amphetamine release from AM-9, reducing the overall area under the curve (AUC).

The test article AM-9 was prepared in reverse osmosis (RO) water with 0.1% formic acid. The formulation was a clear solution. Animals were either fasted overnight through 4 hours postdose or fed ad lib prior to dosing, 4 animals per groups. Individual doses of AM-9 was calculated based on body weights taken on the day of dosing. The liquid formulation was administered via oral gavage at 10 mg/kg. Prior to withdrawing the gavage tube, the tube was flushed with approximately 2 mL of water.

Blood was collected from a jugular vein into tubes containing K₂EDTA anticoagulant. Blood (approximately 0.5 mL) was collected from each animal predose and at 0.167, 0.333, 0.5, 0.75, 1, 2, 4,5, and 8 hours postdose and processed for plasma. Plasma samples were evaluated for amphetamine by LC-MS-MS Mass spectrometer (API 5000 AB Sciex Instruments).

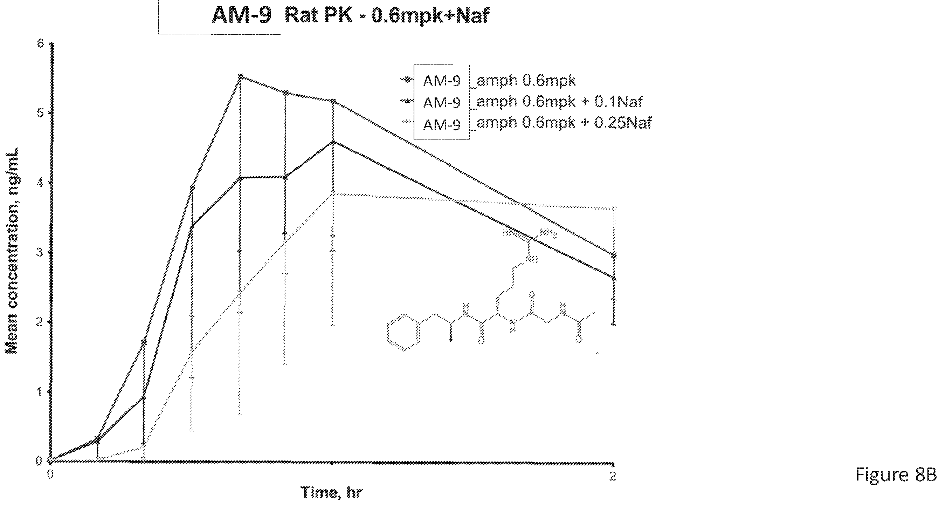

Figure 8A depicts the mean plasma concentration of amphetamine over 8 hours after administration of Compound AM-9 at doses of 0.6 mg/kg alone and in combination with 0.1 mg/kg of Nafamostat and 0.25 mg/kg of Nafamostat. Figure 8B depicts the mean plasma concentration of amphetamine over the first 2 hours after administration of Compound AM-9 at doses of 0.6 mg/kg alone and in combination with 0.1 mg/kg of Nafamostat and 0.25 mg/kg of Nafamostat. As demonstrated by Figures 8A-8B, a trypsin inhibitor reduces the release of amphetamine from Compound AM-9 from oral administration.

### Example 9: Pharmacokinetics of Compound AM-9 following PO administration to rats without and with trypsin inhibitor Nafamostat.

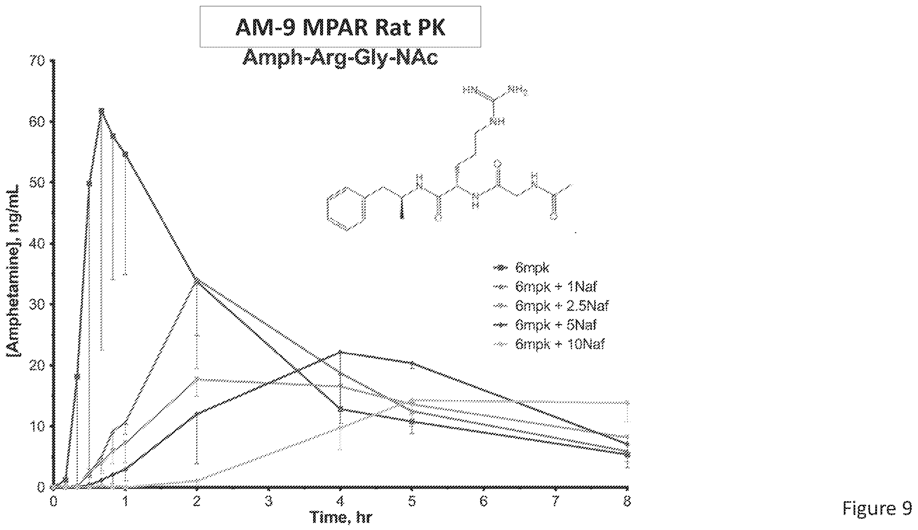

Figure 9 compares mean plasma concentrations over time of amphetamine following PO administration of Compound AM-9 to rats in the absence and presence of trypsin inhibitor nafamostat. This example provides a comparison of the immediate release of amphetamine into plasma when Compound AM-9 at a dosage of 6 mg/kg is administered PO to fasted rats alone, or with 1, 2.5, 5 and 10 mg/kg trypsin inhibitor Nafamostat. The data shows that Nafamosta reduces the Cmax and increases the Tmax for the amphetamine release from AM-9, reducing the overall area under the curve (AUC).

Compound AM-9 was prepared in reverse osmosis (RO) water with 0.1% formic acid. The formulation was a clear solution. Animals were either fasted overnight through 4 hours postdose or fed ad lib prior to dosing, 4 animals per groups. Individual doses of AM-9 was calculated based on body weights taken on the day of dosing. The liquid formulation was administered via oral gavage at 10 mg/kg. Prior to withdrawing the gavage tube, the tube was flushed with approximately 2 mL of water.

Blood was collected from a jugular vein into tubes containing K₂EDTA anticoagulant. Blood (approximately 0.5 mL) was collected from each animal predose and at 0.167, 0.333, 0.5, 0.75, 1, 2, 4, 5, and 8 hours postdose and processed for plasma. Plasma samples were evaluated for amphetamine by LC-MS-MS Mass spectrometer (API 5000 AB Sciex Instruments).

### Example 10: Pharmacokinetics of Compound AM-9 and amphetamine following intravenous (IV) administration of Compound AM-9 to rats

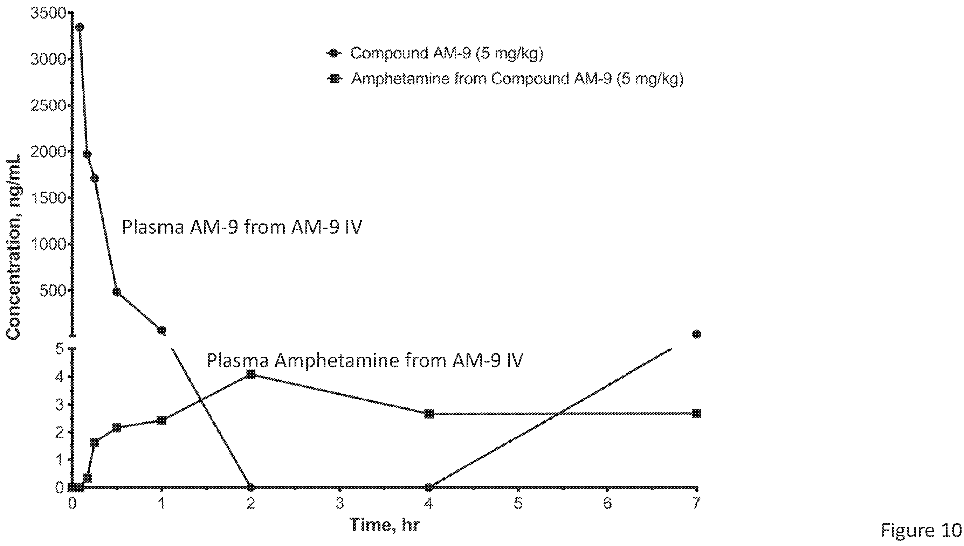

Figure 10 compares mean plasma concentrations over time of Compound AM-9 and the resulting mean plasma concentrations of amphetamine over time following IV administration of Compound AM-9 to rats. This example depicts the mean plasma concentrations following IV administration of Compound AM-9 to rats at doses of 5 mg/kg. This example demonstrates that there is very little conversion of AM-9 to amphetamine in the blood.

Compound AM-9 was prepared in reverse osmosis (RO) water with 0.1% formic acid. The formulation was a clear solution. Animals were fasted overnight through 4 hours postdose prior to dosing, 4 animals per groups. Individual doses of AM-9 was calculated based on body weights taken on the day of dosing. The liquid formulation was administered IV at 5 mg/kg of Compound AM-9.

Blood was collected from a jugular vein into tubes containing K₂EDTA anticoagulant. Blood (approximately 1 mL) was collected from each animal predose and at 0.167, 0.333, 0.5, 0.667, 0.833 1, 2, 4, and 7 hours postdose and processed for plasma. Plasma samples were evaluated for either AM-9 or amphetamine by LC-MS-MS Mass spectrometer (API 5000 AB Sciex Instruments).

### Example 11: Pharmacokinetics of Compound AM-9 in plasma and cererbral spinal fluid (CSF) following intravenous (IV) administration of Compound AM-9 to rats

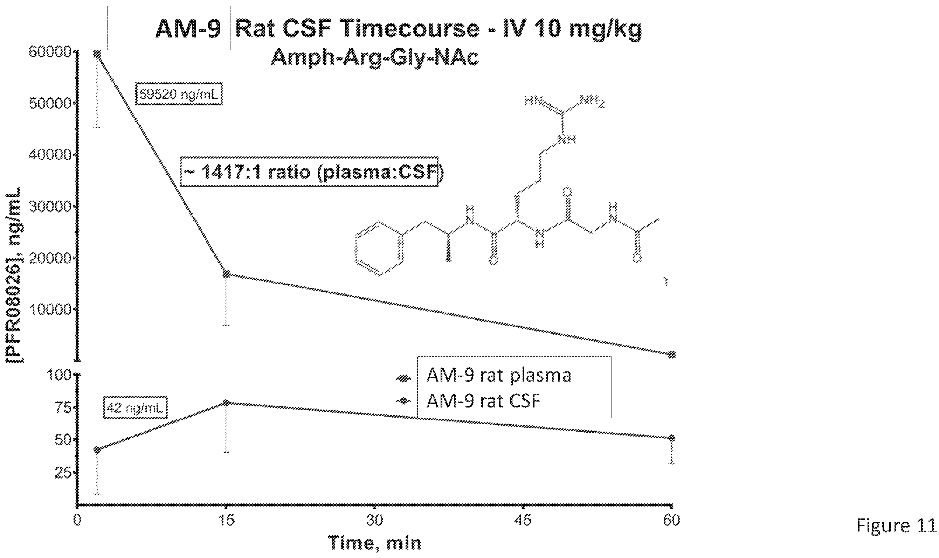

Figure 11 compares mean plasma concentrations over time and the mean CSF concentrations over time of Compound AM-9 following IV administration of Compound AM-9 to rats. This example depicts the mean plasma concentrations following IV administration of Compound AM-9 to rats at doses of 10 mg/kg mg/kg. This example demonstrates that there is very little AM-9 that crosses from plasma into the CSF.

Compound AM-9 was prepared in reverse osmosis (RO) water with 0.1% formic acid. The formulation was a clear solution. Animals were fasted overnight through 4 hours postdose prior to dosing, 4 animals per groups. Individual doses of AM-9 was calculated based on body weights taken on the day of dosing. The liquid formulation was administered IV at 10 mg/kg of Compound AM-9.

Blood was collected from a jugular vein into tubes containing K₂EDTA anticoagulant. Blood (approximately 1 mL) was collected from each animal predose and at 2, 15 and 60 min postdose and processed for plasma. CSF was obtained at these same time periods. Plasma and CSF samples were evaluated for AM-9 by LC-MS-MS Mass spectrometer (API 5000 AB Sciex Instruments).

### Example 12: Pharmacokinetics of Compound AM-10 following oral (PO) administration to rats

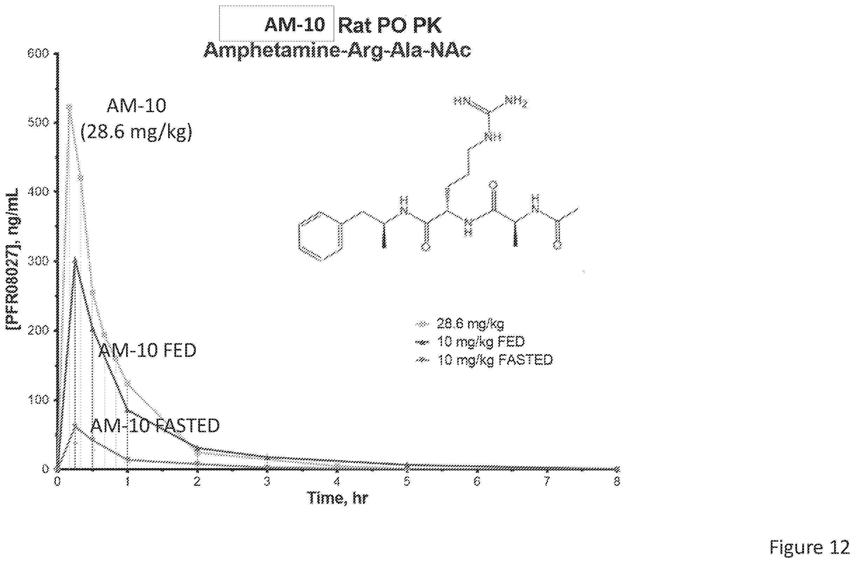

Figure 12 compares mean plasma concentrations over time of AM-10 following PO administration of Compound AM-10 to rats. This example provides a comparison of absorption into plasma when Compound AM-10 at a dosage of 28.6 and 10 mg/kg is administered PO to fasted rats and when 10 mg/kg is administered to fed rats.

Compound AM-10 was prepared in reverse osmosis (RO) water with 0.1% formic acid. The formulation was a clear solution. Animals were either fasted overnight through 4 hours postdose or fed ad lib prior to dosing, 4 animals per groups. Individual doses of AM-10 was calculated based on body weights taken on the day of dosing. The liquid formulation was administered via oral gavage at 10 mg/kg. Prior to withdrawing the gavage tube, the tube was flushed with approximately 2 mL of water.

Blood was collected from a jugular vein into tubes containing K₂EDTA anticoagulant. Blood (approximately 0.5 mL) was collected from each animal predose and at 0.25, 0.5, 1, 2, 3, 5, and 8 hours postdose and processed for plasma. Plasma samples were evaluated for AM-10 by LC-MS-MS Mass spectrometer (API 5000 AB Sciex Instruments).

### Example 13: Pharmacokinetics of Amphetamin release Compound AM-10 following PO administration to rats

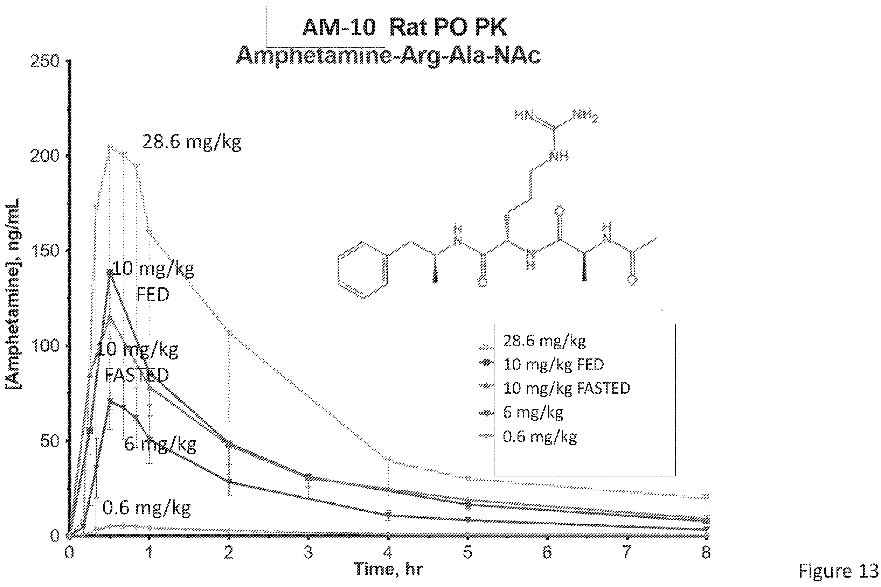

Figure 13 compares mean plasma concentrations over time of amphetamine release following PO administration of different doses of Compound AM-10 to rats. This example depicts the mean plasma concentrations following PO administration to rats at doses of 28.6mg/kg, 10 mg/kg, 6 mg/kg and 0.6 mg/kg of Compound AM-10. This example provides a comparison of the immediate release of amphetamine into plasma when Compound AM-9 at a dosage of 10 mg/kg is administered PO to fasted rats and fed rats.

Compound AM-9 was prepared in reverse osmosis (RO) water with 0.1% formic acid. The formulation was a clear solution. Animals were either fasted overnight through 4 hours postdose or fed ad lib prior to dosing, 4 animals per groups. Individual doses of AM-9 was calculated based on body weights taken on the day of dosing. The liquid formulation was administered via oral gavage at 10 mg/kg. Prior to withdrawing the gavage tube, the tube was flushed with approximately 2 mL of water.

Blood was collected from a jugular vein into tubes containing K₂EDTA anticoagulant. Blood (approximately 0.5 mL) was collected from each animal predose and at 0.25, 0.5, 1, 2, 3, 5, and 8 hours postdose and processed for plasma. Plasma samples were evaluated for amphetamine by LC-MS-MS Mass spectrometer (API 5000 AB Sciex Instruments).

## Claims

1. Amphetamine-arginine-glycine-acetate, Compound AM-9, shown below: 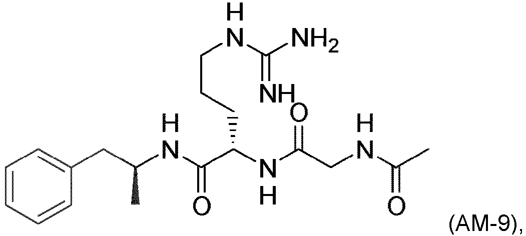 or acceptable salts, solvates, and hydrates thereof.

2. The compound of Claim 1 for use in medical therapy.

3. The compound of Claim 1 for use in the treatment or prevention of attention deficit hyperactivity disorder (ADHD).

4. A composition comprising amphetamine-arginine-glycine-acetate, Compound AM-9, shown below: 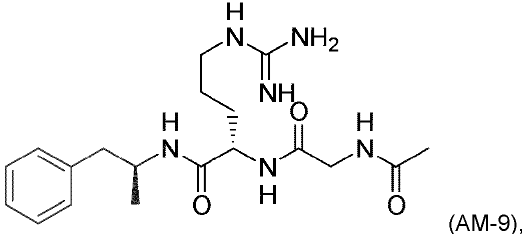 or acceptable salts, solvates, and hydrates thereof.

5. A composition comprising a trypsin inhibitor and amphetamine-arginine-glycine-acetate, Compound AM-9, shown below: 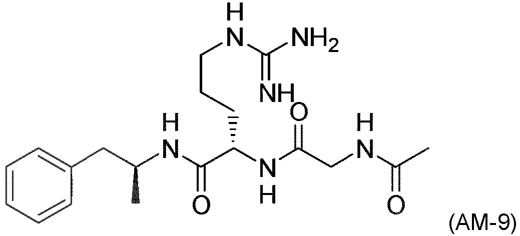 or acceptable salts, solvates, and hydrates thereof.

6. The composition of Claim 5, wherein the trypsin inhibitor comprises 6-carbamimidoylnaphthalen-2-yl 4-(diaminomethyleneamino)benzoate: 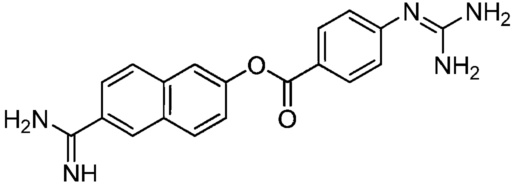 or a pharmaceutically acceptable salt thereof.

7. The composition of any one of Claims 4-6, for use in the treatment or prevention of attention deficit hyperactivity disorder (ADHD).

8. Amphetamine-arginine-alanine-acetate, Compound AM-10, shown below: 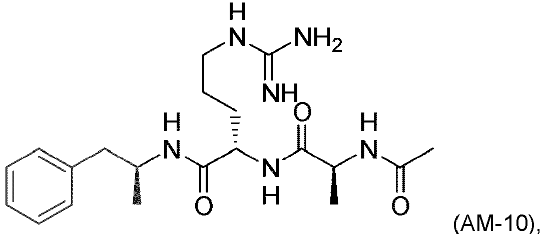 or acceptable salts, solvates, and hydrates thereof.

9. The compound of Claim 8 for use in medical therapy.

10. The compound of Claim 8 for use in the treatment or prevention of attention deficit hyperactivity disorder (ADHD).

11. A composition comprising amphetamine-arginine-alanine-acetate, Compound AM-10, shown below: 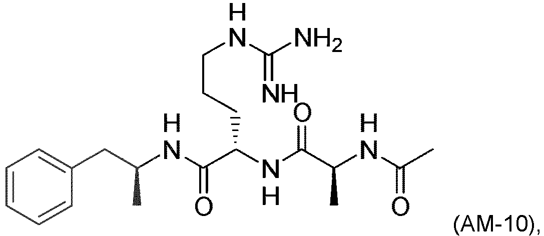 or acceptable salts, solvates, and hydrates thereof.

12. A composition comprising a trypsin inhibitor and amphetamine-arginine-alanine-acetate, Compound AM-10, shown below: 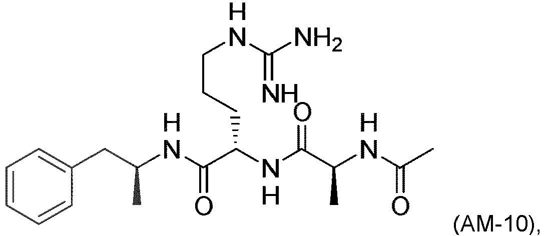 or acceptable salts, solvates, and hydrates thereof.

13. The composition of Claim 12, wherein the trypsin inhibitor comprises 6-carbamimidoylnaphthalen-2-yl 4-(diaminomethyleneamino)benzoate: 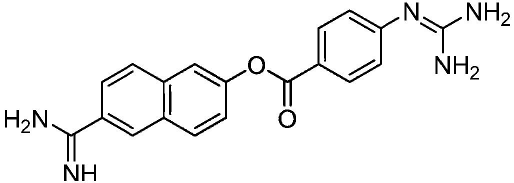 or a pharmaceutically acceptable salt thereof.

14. The composition of any one of Claims 11-13 for use in the treatment or prevention of attention deficit hyperactivity disorder (ADHD).

## Patentansprüche

1. Amphetaminargininglycinacetat, Verbindung AM-9, die nachfolgend dargestellt ist: 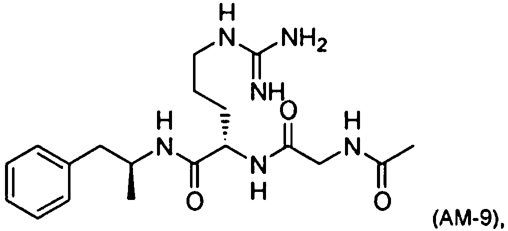 oder annehmbare Salze, Solvate und Hydrate davon.

2. Verbindung nach Anspruch 1 zur Verwendung in der medizinischen Therapie.

3. Verbindung nach Anspruch 1 zur Verwendung bei der Behandlung oder Prävention einer Aufmerksamkeitsdefizit-Hyperaktivitätsstörung (ADHS).

4. Zusammensetzung, die Amphetaminargininglycinacetat, Verbindung AM-9, umfasst, die nachfolgend dargestellt ist: 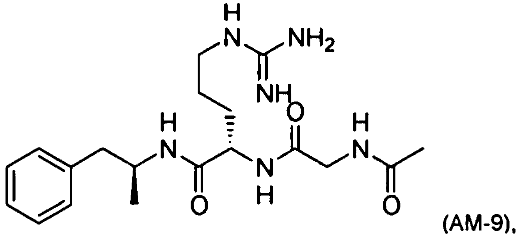 oder annehmbare Salze, Solvate und Hydrate davon.

5. Zusammensetzung, die einen Trypsininhibitor und Aphetaminargininglycinacetat, Verbindung AM-9, umfasst, die nachstehend dargestellt ist: 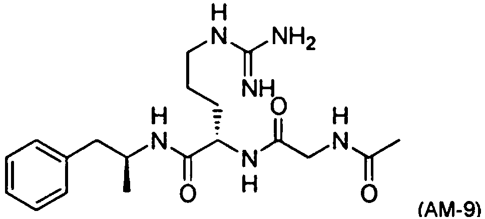 oder annehmbare Salze, Solvate und Hydrate davon.

6. Zusammensetzung nach Anspruch 5, wobei der Trypsininhibitor 6-Carbamimidoyl-naphthalin-2-yl-4-(diaminomethylenamino)benzoat: 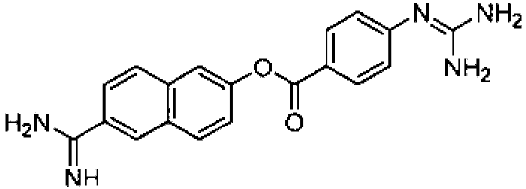 oder ein pharmazeutisch annehmbares Salz davon umfasst.

7. Zusammensetzung nach einem der Ansprüche 4 bis 6 zur Verwendung bei der Behandlung oder Prävention einer Aufmerksamkeitsdefizit-Hyperaktivitätsstörung (ADHS).

8. Amphetaminargininalaninacetat, Verbindung AM-10, die nachstehend dargestellt ist: 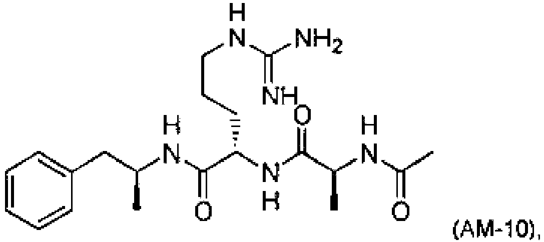 oder annehmbare Salze, Solvate und Hydrate davon.

9. Verbindung nach Anspruch 8 zur Verwendung in der medizinischen Therapie.

10. Verbindung nach Anspruch 8 zur Verwendung bei der Behandlung oder Prävention einer Aufmerksamkeitsdefizit-Hyperaktivitätsstörung (ADHS).

11. Zusammensetzung, die Amphetaminargininalaninacetat, Verbindung AM-10, umfasst, die nachstehend dargestellt ist: 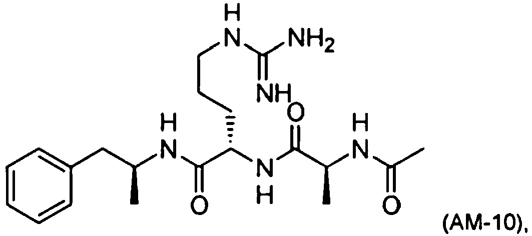 oder annehmbare Salze, Solvate und Hydrate davon.

12. Zusammensetzung, die umfassend einen Trypsininhibitor und Amphetaminargininalaninacetat, Verbindung AM-10, umfasst, die nachstehend dargestellt ist: 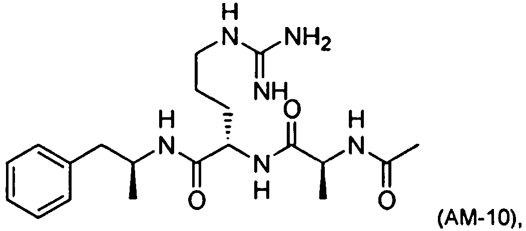 oder annehmbare Salze, Solvate und Hydrate davon.

13. Zusammensetzung nach Anspruch 12, wobei der Trypsininhibitor Carbamimidoyl-naphthalin-2-yl-4-(diaminomethylenamino)benzoat: 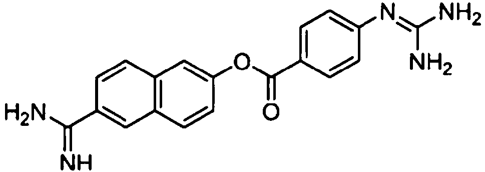 oder ein pharmazeutisch annehmbares Salz davon umfasst.

14. Zusammensetzung nach einem der Ansprüche 11 bis 13 zur Verwendung bei der Behandlung oder Prävention einer Aufmerksamkeitsdefizit-Hyperaktivitätsstörung (ADHS).

## Revendications

1. Amphétamine-arginine-glycine-acétate, composé AM-9, représenté ci-dessous : 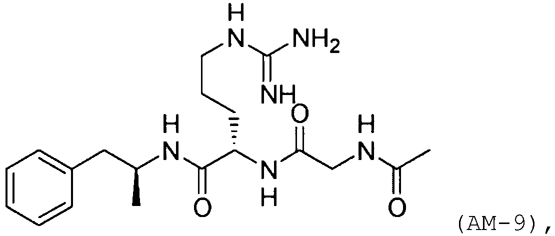 ou des sels, des solvates et des hydrates pharmaceutiquement acceptables de celui-ci,

2. Composé selon la revendication 1 pour utilisation en thérapie médicale.

3. Composé selon la revendication 1 pour utilisation dans le traitement ou la prévention du trouble déficitaire de l'attention avec hyperactivité (TDAH).

4. Composition comprenant de l'amphétamine-arginine-glycine-acétate, composé AM-9, représenté ci-dessous : 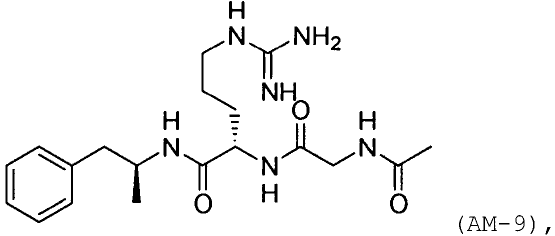 ou des sels, des solvates et des hydrates pharmaceutiquement acceptables de celui-ci,

5. Composition comprenant un inhibiteur de trypsine et de l'amphétamine-arginine-glycine-acétate, composé AM-9, représenté ci-dessous : 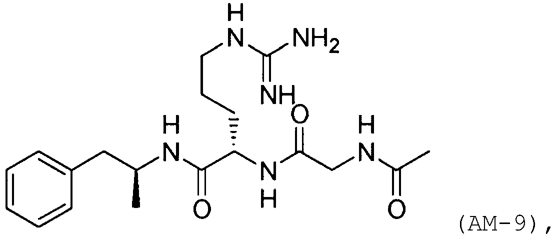 ou des sels, des solvates et des hydrates pharmaceutiquement acceptables de celui-ci,

6. Composition selon la revendication 5, dans laquelle l'inhibiteur de trypsine comprend du 4-(diaminométhylèneamino)benzoate de 6-carbamimidoylnaphtalène-2-yle : 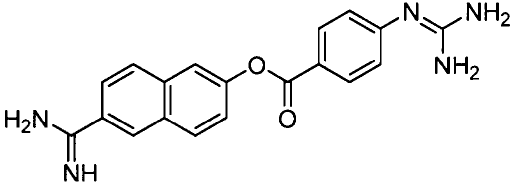 ou un sel pharmaceutiquement acceptable de celui-ci.

7. Composition selon l'une quelconque des revendications 4 à 6 pour utilisation dans le traitement ou la prévention du trouble déficitaire de l'attention avec hyperactivité (TDAH).

8. Amphétamine-arginine-alanine-acétate, composé AM-10, représenté ci-dessous : 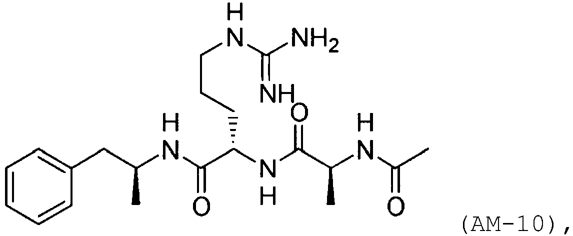 ou des sels, des solvates et des hydrates pharmaceutiquement acceptables de celui-ci,

9. Composé selon la revendication 8 pour utilisation en thérapie médicale.

10. Composé selon la revendication 8 pour utilisation dans le traitement ou la prévention du trouble déficitaire de l'attention avec hyperactivité (TDAH).

11. Composition comprenant de l'amphétamine-arginine-alanine-acétate, composé AM-10, représenté ci-dessous : 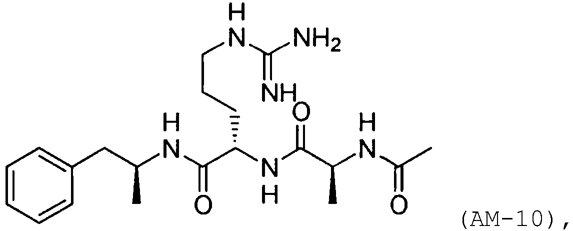 ou des sels, des solvates et des hydrates pharmaceutiquement acceptables de celui-ci,

12. Composition comprenant un inhibiteur de trypsine et de l'acétate d'amphétamine-arginine-alanine, composé AM-10, représenté ci-dessous : 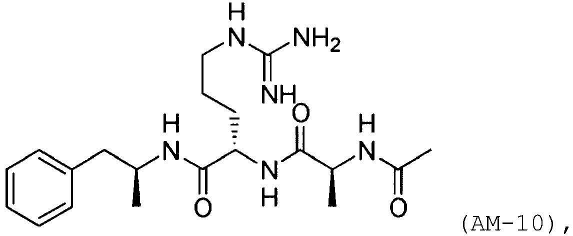 ou des sels, des solvates et des hydrates pharmaceutiquement acceptables de celui-ci,

13. Composition selon la revendication 12, dans laquelle l'inhibiteur de trypsine comprend du 4-(diaminométhylèneamino)benzoate de 6-carbamimidoylnaphtalène-2-yle : 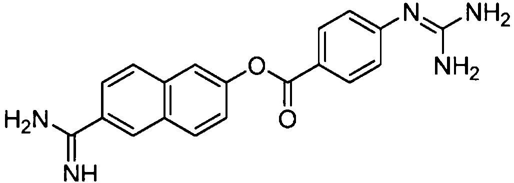 ou un sel pharmaceutiquement acceptable de celui-ci.

14. Composition selon l'une quelconque des revendications 11 à 13 pour utilisation dans le traitement ou la prévention du trouble déficitaire de l'attention avec hyperactivité (TDAH).
